# EUROPEAN PATENT SPECIFICATION

(11) **EP 2 627 199 B1**
(45) Date of publication and mention of the grant of the patent: **19.04.2017**
(21) Application number: 11776954.7
(22) Date of filing: 12.10.2011
(51) Int. Cl.: A61K 8/97, A61K 31/35, A61K 36/74, A23L 29/00

(54) **COFFEE EXTRACTS AS INGREDIENTS OF FOODS, DRUGS, COSMETICS, DIETARY SUPPLEMENTS, AND BIOLOGICS**
KAFFEEEXTRAKTE ALS INHALTSSTOFFE VON NAHRUNGSMITTELN, ARZNEIMITTELN, KOSMETIKA, NAHRUNGSERGÄNZUNGSMITTELN UND BIOLOGIKA.
EXTRAITS DE CAFE COMME INGREDIENTS DE COMPOSITIONS ALIMENTAIRES, PHARMACEUTIQUES, COSMETIQUES, DIETETIQUES ET BIOLOGIQUES

(30) Priority: 13.10.2010 US 392828 P
(43) Date of publication of application: 21.08.2013
(73) Proprietor: Koninklijke Douwe Egberts B.V., 3532 AD Utrecht (NL)
(72) Inventor: CHU, Yi-Fang, Glenview, Illinois 60026 (US); CHEN, Yumin, Gurnee, Illinois 60031 (US); BROWN, Peter H., Glenview, Illinois 60025 (US); LYLE, Barbara J., Deerfield, Illinois 60015 (US)
(74) Representative: Boult Wade Tennant
(86) International application number: PCT/US2011/055950
(87) International publication number: WO 2012/051287

(56) References cited:
- EP-A1- 1 557 097
- EP-A1- 1 674 106
- WO-A1-01/56586
- WO-A1-2010/051120
- WO-A1-2010/051216
- DE-A1- 19 604 030
- JP-A- 2002 087 977

## Description

### FIELD

The field is related to coffee materials or coffee extracts as ingredients of food, drug, cosmetic, dietary supplement, and biologic products, and related processes.

### BACKGROUND

Crude caffeine is a major byproduct of green coffee bean decaffeination. Decaffeinated coffee accounts for about 10% of overall coffee consumption; thus thousands of tons of crude caffeine are produced annually. In addition to caffeine, green coffee beans contain a wide variety of bioactive phytochemicals, including chlorogenic acids (Michael, N. C., Journal of the Science of Food and Agriculture 1999, 79, 362-372) and coffee oils (Araujo, J. M. A. and Sandi, D., Food Chemistry 2007, 101, 1087-1094). The "phyto-" of the word phytochemical is derived from the Greek word phyto, which means plant; therefore, phytochemicals are plant chemicals. Some of the non-caffeine phytochemicals are inevitably removed during the process of decaffeination, giving rise to the yellow-green color of crude caffeine.

Crude caffeine is the starting material for manufacturing pure caffeine, which is used in beverages, foods, and medicines; the non-caffeine residues are typically discarded as waste. In light of the many studies that have demonstrated health benefits of coffee phytochemicals (Svilaas, A. et al., Journal of Nutrition 2004, 134, S62-S67; Daglia, M. et al., Journal of Agricultural and Food Chemistry 2000, 48, 1449-1454), we postulated that crude caffeine may also exert beneficial effects due to the presence of these non-caffeine components.

EP1557097 discloses a process for selectively removing caffeine from a caffeine-containing catechin composition.

WO2010051216 discloses a process to recover an antioxidant component from coffee beans.

DE19604030 discloses the use of coffee beans, especially untreated, dried coffee beans, to obtain an antioxidant.

JP2002087977 discloses a prophylactic, improving and therapeutic agent for hypertension.

WO0156586 discloses a process for the isolation and purification of caffeine-free mixtures catechins from various different biomass sources.

WO2010051120 discloses a pet food composition comprising an antioxidant component.

### SEGUE

We evaluated crude caffeine for phenolic content, hydrophilic and lipophilic oxygen radical absorbance capacity (ORAC_{hydro} and ORACₗᵢₚₒ), protection of mouse primary neuron cells from hydrogen peroxide-induced cell death, anti-hyperglycemic effects in cultured human skeletal muscle cells and adipocytes, and inhibitory action against cyclooxygenase-2 (COX-2), a potent mediator of inflammation. To determine the chemical structures of the bioactive compounds in the crude caffeine, we performed activity-guided fractionation of antioxidant compounds followed by liquid chromatography-mass spectrometry (LC-MS) multiple reaction monitoring (MRM) analysis.

### SUMMARY

A first embodiment is a process for producing a food product comprising either supplementing a food with, or supplementing a component of a food with, a phytochemical fraction recovered from a crude caffeine, whereby said food product is produced; wherein said phytochemical fraction has a ratio of polyphenols to caffeine of about 10 - 40 to 1, wherein said phytochemical fraction is a retentate of a filtration process of a water suspension of crude caffeine, and wherein said crude caffeine is a product of a green coffee bean supercritical carbon dioxide (scCO₂) decaffeination process.

Preferably the ratio of polyphenols to caffeine is about 10 - 30 to 1. Preferably the ratio of polyphenols to caffeine is about 20 to 1.

Preferably the crude caffeine has an oxygen radical absorbance capacity (ORAC_{hydro} and ORACₗᵢₚₒ) greater than pure caffeine. Preferably the crude caffeine has an ORAC_{hydro} of 145 mmol TE/g and an ORACₗᵢₚₒ of 66 mmol TE/g. Preferably the crude caffeine comprises about 1 wt. % phenolics.

Preferably the phytochemical fraction comprises a water-insoluble particles (WIP) fraction of crude caffeine comprising caffeic acid, coumaric acid, vanillic acid, quercetin, catechin, abscisic acid glucose ester, and threonine.

Preferably the food product comprises alpha-tocopherol and one of a water-insoluble particles (WIP) fraction of crude caffeine, catechin, and chlorogenic acid.

A second embodiment is a composition of matter comprising a food product, said food product comprised of a phytochemical fraction recovered from a crude caffeine, said phytochemical fraction having a ratio of polyphenols to caffeine of about 10 - 40 to 1, wherein said phytochemical fraction is obtainable as a retentate of a filtration process of a water suspension of crude caffeine, and wherein said crude caffeine is a product of a green coffee bean supercritical carbon dioxide (scCO₂ decaffeination process.
Preferably the ratio of polyphenols to caffeine is about 10 - 30 to 1. Preferably the ratio of polyphenols to caffeine is about 20 to 1.
Preferably the crude caffeine has an oxygen radical absorbance capacity (ORAC_{hydro} and ORACₗᵢₚₒ) greater than pure caffeine. Preferably the crude caffeine has an ORAC_{hydro} of 145 mmol TE/g and an ORACₗᵢₚₒ of 66 mmol TE/g. Preferably the crude caffeine comprises about 1 wt. % phenolics.
Preferably the process comprises forming a suspension of the crude caffeine and collecting water-insoluble particles (WIP) of the crude caffeine by filtration. Preferably the WIP comprises caffeic acid, coumaric acid, vanillic acid, quercetin, catechin, abscisic acid glucose ester, and threonine.

A third embodiment is a composition of matter comprising a food product as defined in above, for use in the treatment of diabetes mellitus, inhibition of inflammation, dementia, osteoarthritis, rheumatoid arthritis, or age related cognitive decline.
There is also described herein a composition of matter comprising a food product, said food product comprised of a phytochemical fraction recovered from a crude caffeine, said phytochemical fraction having a ratio of polyphenols to caffeine of about 20, 10-30 or 40, or greater than 10. There is also described herein wherein said phytochemical fraction is a retentate of a filtration process of a water suspension of crude caffeine, and wherein said crude caffeine is a product of a green coffee bean decaffeination process.
There is also described herein a process for producing a food product comprising either supplementing a food with, or supplementing a component of a food with, a phytochemical fraction recovered from a crude caffeine, said phytochemical fraction being that as described in the first embodiment or its related embodiment, whereby said food product is produced.

There is also described herein a composition of matter comprising a drug product, said drug product comprised of a phytochemical fraction recovered from a crude caffeine, said phytochemical fraction having a ratio of polyphenols to caffeine of about 20, 10-30 or 40, or greater than 10. There is also described herein wherein said phytochemical fraction is a retentate of a filtration process of a water suspension of crude caffeine, and wherein said crude caffeine is a product of a green coffee bean decaffeination process.

There is also described herein a process for producing a drug product comprising either supplementing a drug with, or supplementing a component of a drug with, a phytochemical fraction recovered from a crude caffeine, said phytochemical fraction being that as described herein, whereby said drug product is produced.

There is also described herein a composition of matter comprising a cosmetic product, said cosmetic product comprised of a phytochemical fraction recovered from a crude caffeine, said phytochemical fraction having a ratio of polyphenols to caffeine of about 20, 10-30 or 40, or greater than 10. There is also described herein wherein said phytochemical fraction is a retentate of a filtration process of a water suspension of crude caffeine, and wherein said crude caffeine is a product of a green coffee bean decaffeination process.

There is also described herein a process for producing a cosmetic product comprising either supplementing a cosmetic with, or supplementing a component of a cosmetic with, a phytochemical fraction recovered from a crude caffeine, said phytochemical fraction being that as described herein, whereby said cosmetic product is produced.

There is also described herein a composition of matter comprising a dietary supplement product, said dietary supplement product comprised of a phytochemical fraction recovered from a crude caffeine, said phytochemical fraction having a ratio of polyphenols to caffeine of about 20, 10-30 or 40, or greater than 10. There is also described herein wherein said phytochemical fraction is a retentate of a filtration process of a water suspension of crude caffeine, and wherein said crude caffeine is a product of a green coffee bean decaffeination process.

There is also described herein a process for producing a dietary supplement product comprising either supplementing a dietary supplement with, or supplementing a component of a dietary supplement with, a phytochemical fraction recovered from a crude caffeine, said phytochemical fraction being that as described herein, whereby said dietary supplement product is produced.

There is also described herein a composition of matter comprising a biologic product, said biologic product comprised of a phytochemical fraction recovered from a crude caffeine, said phytochemical fraction having a ratio of polyphenols to caffeine of about 20, 10-30 or 40, or greater than 10. There is also described herein wherein said phytochemical fraction is a retentate of a filtration process of a water suspension of crude caffeine, and wherein said crude caffeine is a product of a green coffee bean decaffeination process.

There is also described herein a process for producing a biologic product comprising either supplementing a biologic with, or supplementing a component of a biologic with, a phytochemical fraction recovered from a crude caffeine, said phytochemical fraction being that as described in the fifth embodiment or its related embodiment, whereby said biologic product is produced.

There is also described herein a process for facilitating neuroprotection, said process comprising: identifying a human subject in need of neuroprotection; and administering an amount of the composition described herein to said subject effective to facilitate neuroprotection. There is also described herein a process for facilitating neuroprotection, said process comprising: administering an amount of the described herein to a human subject effective to facilitate neuroprotection; and measuring neuroprotection in said subject.

There is also described herein a process for inhibiting COX-2, said process comprising: identifying a human subject in need of inhibition of COX-2; and administering an amount of the composition described herein to said subject effective to inhibit COX-2. There is also described herein a process for inhibiting COX-2, said process comprising: administering an amount of the composition described herein to a human subject effective to inhibit COX-2; and measuring inhibition of COX-2 in said subject.

There is also described herein a process for stimulating glucose uptake, said process comprising: identifying a human subject in need of stimulation of glucose uptake; and administering an amount of the composition described herein, to said subject effective to stimulate glucose uptake. There is also described herein a process for stimulating glucose uptake, said process comprising: administering an amount of the composition described herein, to a human subject effective to stimulate glucose uptake; and measuring stimulation of glucose uptake in said subject.

There is also described herein a process for treating dementia or age related cognitive decline, said process comprising: identifying a human subject in need of treatment of dementia or age related cognitive decline; and administering an amount of the composition described herein to said subject effective to treat dementia or age related cognitive decline.

There is also described herein a process for treating dementia or age related cognitive decline, said process comprising: administering an amount of the composition described herein to a human subject effective to treat dementia or age related cognitive decline; and measuring treatment of dementia or age related cognitive decline in said subject.

There is also described herein a process for inhibiting inflammation, said process comprising: identifying a human subject in need of inhibition of inflammation; and administering an amount of the composition described herein to said subject effective to inhibit inflammation. There is also described herein a process for inhibiting inflammation, said process comprising: administering an amount of the composition described herein to a human subject effective to inhibit inflammation; and measuring inhibition of inflammation in said subject.

There is also described herein a process for treating diabetes mellitus, said process comprising: identifying a human subject in need of treatment of diabetes mellitus; and administering an amount of the composition described herein to said subject effective to treat diabetes mellitus. There is also described herein a process for treating diabetes mellitus, said process comprising: administering an amount of the composition described herein to a human subject effective to treat diabetes mellitus; and measuring treatment of diabetes mellitus in said subject.

There is also described herein, wherein the identifying step or the measuring step is by neuropsychological testing. There is also described herein wherein the identifying step or the measuring step is by rheumatology criteria. There is also described herein wherein the identifying step or the measuring step is by blood glucose concentration.

### DRAWINGS

FIG. 1. Flow diagram of crude caffeine being further processed to reduce caffeine and enrich polyphenols having a food product as an end product.
FIG. 2. Flow diagram of crude caffeine being further processed to reduce caffeine and enrich polyphenols having a drug product as an end product.
FIG. 3. Flow diagram of crude caffeine being further processed to reduce caffeine and enrich polyphenols having a cosmetic product as an end product.
FIG. 4. Flow diagram of crude caffeine being further processed to reduce caffeine and enrich polyphenols having a dietary supplement product as an end product.
FIG. 5. Flow diagram of crude caffeine being further processed to reduce caffeine and enrich polyphenols having a biologic product as an end product.
FIG. 6. Bioactive compounds of crude caffeine obtained during coffee production.
FIG. 7. Supercritical CO₂ extraction of caffeine from coffee.
FIG. 8. Effect of pure caffeine (A) or crude caffeine (B) on hydrogen peroxide-treated mouse primary neuron cells.
FIG. 9. Stimulation of glucose uptake in human skeletal muscle cells and adipocytes by insulin, crude caffeine, and pure caffeine.
FIG. 10. Inhibition of COX-2 by aspirin, regular coffee, pure caffeine, and crude caffeine.
FIG. 11. Isolation of water-insoluble particles from crude caffeine.
FIG. 12. Water-insoluble particles protect mouse primary neuron cells against hydrogen peroxide-induced cell death.
FIG. 13. Liquid chromatography-mass spectrometry identification of chemicals in water-insoluble particles with the multiple reaction monitoring technique. (A) Caffeic acid; (B) Coumaric acid; (C) Vanillic acid; (D) Quercetin; (E) Catechin; (F) Abscisic acid glucose ester; (G) Threonine.

### DETAILED DESCRIPTION

Thousands of tons of crude caffeine are obtained annually during coffee decaffeination, and phytochemicals are removed along with the caffeine. We determined the following characteristics of crude caffeine produced by supercritical carbon dioxide (scCO₂) decaffeination: phenolic content, hydrophilic and lipophilic oxygen radical absorbance capacities (ORAC_{hydro} and ORACₗᵢₚₒ), protection of neuron cells from hydrogen peroxide-induced cell death, and ability to simulate glucose uptake and inhibit cyclooxygenase-2 (COX-2). Crude caffeine contained 10 mg CE/g phenolics and had an ORAC_{hydro} of 145 µmol TE/g and an ORACₗᵢₚₒ of 66 µmol TE/g, but did not increase survival of hydrogen peroxide-treated primary neuron cells. Crude caffeine increased glucose uptake 1,45-fold in cultured human skeletal muscle cells and 2.20-fold in cultured human adipocytes. Further, crude caffeine exerted a stronger inhibitory effect against COX-2 (IC₅₀, 20 µg/mL) than aspirin (IC₅₀, 190 µg/mL). Pure caffeine did not stimulate glucose uptake or inhibit COX-2. We devised an enrichment method to isolate antioxidants from crude caffeine as water insoluble particles (WIP), which protected primary neuronal cells from hydrogen peroxide-induced cell death in a dose-dependent manner. ORAC-guided fractionation of WIP coupled with LC-MS/MS analysis identified seven bioactive components: caffeic acid, coumaric acid, vanillic acid, quercetin, catechin, abscisic acid glucose ester, and threonine. We discovered that WIP combined with α-tocopherol showed synergistic effects.

### Definitions

Unless defined otherwise, terms used herein have the same meaning as commonly understood by one of ordinary skill in the art to which this invention belongs.

The term "food" means, as stated in the Federal Food, Drug, and Cosmetic (FD&C) Act, (1) an article used for food or drink for man or other animal, (2) chewing gum, and (3) an article used for a component of any such article.

The term "drug" means, as stated in the Federal Food, Drug, and Cosmetic Act, an article recognized in the official United States Pharmacopoeia, official Homoeopathic Pharmacopoeia of the United States, or official National Formulary, or any supplement to any of them, an article intended for use in the diagnosis, cure, mitigation, treatment, or prevention of disease in man or other animal, an article (other than food) intended to affect the structure or any function of the body of man or other animal, and an article intended for use as a component of any article just specified.

The term "cosmetic" means, as stated in the Federal Food, Drug, and Cosmetic Act, an article intended to be rubbed, poured, sprinkled, or sprayed on, introduced into, or otherwise applied to the human body or any part thereof for cleansing, beautifying, promoting attractiveness, or altering the appearance, and an article intended for use as a component of any such article.

The term "dietary supplement" means, as stated in the Federal Food, Drug, and Cosmetic Act, a product intended to supplement the diet that bears or contains one or more of the following dietary ingredients: a vitamin, a mineral, an herb or other botanical, an amino acid, a dietary substance for use by man to supplement the diet by increasing the total dietary intake, or a concentrate, metabolite, constituent, extract, or combination of any ingredient just described.

The term "biologic" means, as defined by the Food and Drug Administration (FDA), a subset of drugs that are distinguished by the biological manufacturing process.

These definitions serve to differentiate a drug substance from a drug product. A drug product is the finished form, e.g., an oral solid dosage form drug product containing the drug substance. Efficacy studies are done to provide evidence of a drug's ability in diagnosis, cure, mitigation, treatment, or prevention of a disease. Diagnosis, cure, mitigation, treatment, or prevention does not mean 100% efficacy. Rather, diagnosis, cure, mitigation, treatment, or prevention means some level of efficacy, anywhere from 1% to 100%.

Food encompasses the following general food categories, as defined by the Food and Drug Administration (FDA): baked goods and baking mixes, including all ready-to-eat and ready-to-bake products, flours, and mixes requiring preparation before serving; beverages, alcoholic, including malt beverages, wines, distilled liquors, and cocktail mix; beverages and beverage bases, nonalcoholic, including only special or spiced teas, soft drinks, coffee substitutes, and fruit and vegetable flavored gelatin drinks; breakfast cereals, including ready-to-eat and instant and regular hot cereals; cheeses, including curd and whey cheeses, cream, natural, grating, processed, spread, dip, and miscellaneous cheeses; chewing gum, including all forms; coffee and tea, including regular, decaffeinated, and instant types; condiments and relishes, including plain seasoning sauces and spreads, olives, pickles, and relishes, but not spices or herbs; confections and frostings, including candy and flavored frosting, marshmallows, baking chocolate, and brown, lump, rock, maple, powdered, and raw sugars; dairy product analogs, including nondairy milk, frozen or liquid creamers, coffee whiteners, toppings, and other nondairy products; egg products, including liquid, frozen, or dried eggs, and egg dishes made therefrom, *i.e.,* egg roll, egg foo young, egg salad, and frozen multicourse egg meals, but not fresh eggs; fats and oils, including margarine, dressings for salads, butter, salad oils, shortenings and cooking oils; fish products, including all prepared main dishes, salads, appetizers, frozen multicourse meals, and spreads containing fish, shellfish, and other aquatic animals, but not fresh fish; fresh eggs, including cooked eggs and egg dishes made only from fresh shell eggs; fresh fish, including only fresh and frozen fish, shellfish, and other aquatic animals; fresh fruits and fruit juices, including only raw fruits, citrus, melons, and berries, and home-prepared "ades" and punches made therefrom; fresh meats, including only fresh or home-frozen beef or veal, pork, lamb or mutton and home-prepared fresh meat-containing dishes, salads, appetizers, or sandwich spreads made therefrom; fresh poultry, including only fresh or home-frozen poultry and game birds and home-prepared fresh poultry-containing dishes, salads, appetizers, or sandwich spreads made therefrom; fresh vegetables, tomatoes, and potatoes, including only fresh and home-prepared vegetables; frozen dairy desserts and mixes, including ice cream, ice milks, sherbets, and other frozen dairy desserts and specialties; fruit and water ices, including all frozen fruit and water ices; gelatins, puddings, and fillings, including flavored gelatin desserts, puddings, custards, parfaits, pie fillings, and gelatin base salads; grain products and pastas, including macaroni and noodle products, rice dishes, and frozen multicourse meals, without meat or vegetables; gravies and sauces, including all meat sauces and gravies, and tomato, milk, buttery, and specialty sauces; hard candy and cough drops, including all hard type candies; herbs, seeds, spices, seasonings, blends, extracts, and flavorings, including all natural and artificial spices, blends, and flavors; jams and jellies, home-prepared, including only home-prepared jams, jellies, fruit butters, preserves, and sweet spreads; jams and jellies, commercial, including only commercially processed jams, jellies, fruit butters, preserves, and sweet spreads; meat products, including all meats and meat containing dishes, salads, appetizers, frozen multicourse meat meals, and sandwich ingredients prepared by commercial processing or using commercially processed meats with home preparation; milk, whole and skim, including only whole, lowfat, and skim fluid milks; milk products, including flavored milks and milk drinks, dry milks, toppings, snack dips, spreads, weight control milk beverages, and other milk origin products; nuts and nut products, including whole or shelled tree nuts, peanuts, coconut, and nut and peanut spreads; plant protein products, including the National Academy of Sciences/National Research Council "reconstituted vegetable protein" category, and meat, poultry, and fish substitutes, analogs, and extender products made from plant proteins; poultry products, including all poultry and poultry-containing dishes, salads, appetizers, frozen multicourse poultry meals, and sandwich ingredients prepared by commercial processing or using commercially processed poultry with home preparation; processed fruits and fruit juices, including all commercially processed fruits, citrus, berries, and mixtures; salads, juices and juice punches, concentrates, dilution, "ades", and drink substitutes made therefrom; processed vegetables and vegetable juices, including all commercially processed vegetables, vegetable dishes, frozen muiticourse vegetable meals, and vegetable juices and blends; snack foods, including chips, pretzels, and other novelty snacks; soft candy, including candy bars, chocolates, fudge, mints, and other chewy or nougat candies; soups, home-prepared, including meat, fish, poultry, vegetable, and combination home-prepared soups; soups and soup mixes, including commercially prepared meat, fish, poultry, vegetable, and combination soups and soup mixes; sugar, white, granulated, including only white granulated sugar; sugar substitutes, including granulated, liquid, and tablet sugar substitutes; and sweet sauces, toppings, and syrups, including chocolate, berry, fruit, corn syrup, and maple sweet sauces and toppings.

### Green Coffee Bean Decaffeination

Caffeine is a physiologically active component in coffee, which has been studied intensively. It was discovered by Runge in 1820. The chemical name of this purine is 1.3.7 trimethylaxanthine. It is characterized as needle-shaped crystals with a melting point of 236°C. Coffee beans contain between 0.8 and 2.8% caffeine, depending on species and origin.

In order to minimize caffeine and still keep the desirable attributes of a coffee beverage, quite a number of decaffeination processes have been developed. In order to minimize flavor and aroma losses, the commercial decaffeination of coffee is at present carried out on the green coffee beans before roasting. The different decaffeination procedures can be classified into three major groups: solvent decaffeination, water decaffeination, and supercritical CO₂ decaffeination.

"Decaffeinated coffee" means in the EU countries a maximum caffeine concentration of 0.1% related to the dry mass; in the US, it means less than 3% of the amount initially present in the beans.

Principally all decaffeination processes entail five steps: swelling the raw beans with water in order to solubilize the caffeine-potassium chlorogenate complex, and to make caffeine available for extraction; extracting the caffeine from the beans with a solvent; steam stripping to remove all solvent residues from the beans (if applied); regenerating adsorbents (if applied); and drying the decaffeinated coffee beans to their initial moisture content.

### Supercritical CO₂ Decaffeination

The supercritical fluid extraction concept was first recognized in 1879. Hannay, J.B. & Hogarth, J. (1879) Proc. Roy. Soc. London, 29, 324 reported that solid compounds could be dissolved in supercritical fluids. Almost all food processing applications of supercritical fluid technology employ CO₂ as the solvent. Dense CO₂ is not only a powerful solvent for a wide range of compounds of interest in food processing, but it is relatively inert, inexpensive, non-toxic, recyclable, non-flammable, readily available in high purity and leaves no residues. With a critical point at 31,1°C and 7.58 MPa (75.8 bars), near critical and supercritical CO₂ can be used at temperatures and pressures which are relatively safe, convenient and particularly appropriate for the extraction of heat-labile compounds (Palmer, M.V. & Ting, S.S. (1995) Food Chem., 52, 345-52). The solubility of a compound in a supercritical fluid is dependent on the density of the solvent, as well as on the physicochemical affinity of the solute for the solvent. Dissolved compounds can be recovered either by simply decreasing the pressure or increasing the temperature in order to decrease density, or by adsorption of the compound on an appropriate adsorbent. As a disadvantage of this technology, however, one has to recognize the high initial investment and maintenance costs due to the high pressure operation. The costs are the most important issues in the commercialization.

The application of using compressed CO₂ for decaffeination was first described in the mid-1960s (Zosel, K. (1965) Chem. Abstr., 63, 110456). The process conditions proposed are 70-90°C and 160-220 bar. Three possible methods were suggested: (1) The moistened green beans are mixed with a stream of CO₂ in a pressure vessel. The caffeine diffuses from the beans into the CO₂ which passes into a washing tower, where the caffeine is absorbed in water. After 10 hours recycling, almost all caffeine is dissolved in the washing water, from which it can be separated by distillation. (2) Using the extraction conditions outlined in the first proposal, the caffeine is removed from the CO₂ fluid by passing this through a bed of activated carbon in which the caffeine is adsorbed. (3) A mixture of green coffee beans and activated carbon pellets is introduced into a pressure vessel, in which CO₂ is brought to 90°C and 220 bar. In 5 hours, the caffeine diffuses from the bean through the CO₂ directly on to the activated carbon. After extraction, the coffee beans are separated from the activated carbon pellets by a vibrating sieve.

On the basis of these three methods, there have been quite a number of developments involving process improvements in terms of economy or quality.

Based on Zosel's second process, the CO₂ decaffeination of green moistened coffee beans has been reported to be used commercially by the HAG Company since 1979 in Germany, wherein quantitative extraction of caffeine is achieved with moist supercritical CO₂ (US Patent 3 879 569).

One modified example of Zosel's first process is reported to be demonstrated by the "Schoeller-Bleckmann Plant" (Lack, E., Seidlitz, H. & Toro, P. (1989) In: Proceedings of the 13th ASIC Colloquium (Paipa) pp. 236-245, ASIC, Paris, France). Moistening to the optimum water content of between 40% and 45% is effected by contact with steam, water or a combination of the two. The plant is designed with high pressure extraction columns. The CO₂-containing dissolved caffeine flows into an adsorption column where it is decaffeinated by a countercurrent flow of fresh water. If a decaffeination rate of more than 97% is necessary (as in the US), then, after the washing column the gas stream has to pass an active carbon adsorption column. The application of a cascade of extractors in series guarantees a reasonably constant concentration of caffeine in the wash water.

GB 2235121 also described a process in which green unmoistened coffee beans are dampened in the high pressure reactor with supersaturated (1-2% by weight H₂O) supercritical CO₂. The decaffeination takes place layer by layer throughout the reactor at about 35% bean moisture, which is said to be optimal for the decaffeination process. The water - originating from the caffeine scrubber - is taken for supersaturation and contains coffee oil and coffee flavor. It is also optionally used for a second extraction step of the beans with water, countercurrent to the CO₂ flow.

EP 0482675 published an "intermittent pressure system" in which the moistened beans are held under CO₂ atmosphere in one of three or more extractors for a few minutes up to a few hours at 250 bar and 60°C. The pressure is then quickly released which leads to an expulsion of the water-caffeine solution out of the cells on to the bean surface. After repressurizing the reactor to 250 bar/60°C, CO₂ is circulated through the coffee bed and the decaffeination is completed. The caffeine is absorbed in water in a washing tower and separated by water evaporation. The wet beans are finally treated in a centrifuge, reducing the residual caffeine solution and predrying the beans.

This system has been improved according to EP 0439710. Instead of supercritical CO₂, a CO₂-saturated, caffeine-free, green coffee extract is used for the decaffeination at pressures up to 300 bar and temperatures up to 110°C for from 1 up to several hours. After rapid release of the pressure, the beans are washed with the liquid for up to 2 hours. The CO₂-saturated, caffeine-containing, green coffee extract is then decaffeinated in a washing tower with supercritical CO₂. From the CO₂, caffeine is separated in a water absorption tower.

Experiments have also been published that used supercritical nitrous oxide, instead of CO₂, as a solvent for decaffeination (Brunner, G. (1987) In; Proceedings of the 12th ASIC Colloquium (Montreux) pp. 294-305, ASIC, Paris, France). It has higher solvent power than CO₂, due to a higher density for a given temperature and pressure conditions, than CO₂, and a relatively low critical temperature (36.5°C). Nitrous oxide can decompose uncontrolled, if handled inappropriately: yet in the temperature range suitable for decaffeination, nitrous oxide can be safely handled, if ignition sources are avoided.

Quantitative information on the adsorption capacity from activated charcoal, which is dependent on the specific surface area, has been reported (Gabel, P.W., Sarge, S. & Camenga, H.K. (1987) In: Proceedings of the 12th ASIC Colloquium (Montreux) pp. 306-312, ASIC, Paris, France). With the calculation of a "caffeine specific surface area" and experimental analysis of the heat of adsorption (which decreases with increasing mass of caffeine, that is with increasing surface coverage), the adsorption capacity can be predicted.

For application in coffee producing countries, Quijano-Rico, M. (1987) In: Proceedings of the 12th ASIC Colloquium (Montreux) pp. 187-193, ASIC, Paris, France proposed the decaffeination of fresh green coffee beans as a tailored modern technology. After harvest, wet preparation and deparchment, the green beans can be decaffeinated. Avoidance of the traditional moistening and drying steps leads to reduced operation costs and improved cup quality. The decaffeination itself can be realized with supercritical CO₂, using a temperature programmed extraction (a continuous increase from 60°C to 85°C, thus reducing the "thermal stress") (DE 3445502).

The first semi-continuously operating process was proposed by US Patent 4 911 941. The essentially caffeine-free supercritical CO₂ is continuously fed to one end of a vertical cylindrical extraction vessel containing green coffee, and the caffeine-laden CO₂ is continuously withdrawn from the opposite end. The moisturized coffee beans are charged under pressure (300 bar) to the extraction vessel via a big ball valve and lock hopper arrangement (US Patent 4 820 537). The decaffeinated beans at the bottom of the vessel are discharged to the bottom lock hopper by opening the ball valves one after another. The valves are then closed and the extraction continues. The caffeine is washed from the CO₂ stream in a countercurrent water wash column, taking advantage here of a favorable distribution coefficient for caffeine to partition into the aqueous phase. The rich caffeine-laden water from the absorber is concentrated by reverse osmosis to obtain caffeine of 97% or greater purity and a permeate containing acidic non-caffeine solids which are added to the process to improve yield and increase the rate of extraction.

An improved CO₂ decaffeination process design is based upon pilot plant data for commercial-scale coffee decaffeination using a special supercritical CO₂ extraction process (Linnig, D.A., Leyers, W.E. & Novak, R.A. (1991) In: Proceedings of the 14th ASIC Colloquium (San Francisco), pp. 357-64, ASIC, Paris, France). It is a high pressure extraction vessel design, of which several columns are continuously passed through countercurrently by CO₂ at 14-35 MPa and 70-130°C for 6-12 hours. The rich CO₂ fluid, laden with caffeine, is then washed with water at reduced temperature (15-50°C) and pressure (5-10 MPA). The aqueous stream containing crude caffeine is sold for further processing (caffeine recovery and refining).

A special apparatus for extracting certain substances from natural products (such as caffeine from tea or coffee) by means of supercritical CO₂ has been proposed by US Patent 5 153 015. The design includes a cylindrical high pressure vessel with annular cylindrical perforated baskets, which contain the natural product and the adsorbent wherein the fluid flows from the outer cylindrical area to the inner one. The pressure drop and the risk of clogging are reduced, the radial increasing velocity improves the mass transfer in the adsorber and the equipment is extremely compact.

A method for producing decaffeinated green coffee beans after pretreatment with an aqueous acid solution has been described by WO92/03061, EP-547119. The acidification, preferably with 1.5-2% citric acid, compensates for the slight loss of acidity which occurs during supercritical decaffeination, thus improving the flavor and aroma of the coffee beverage. The acidifying treatment can be executed in combination with the moistening step before decaffeination.

The decaffeination of coffee beans has been taken as an example for the application of supercritical fluid extraction with CO₂ (McCoy, B.J. (1993) In: Proceedings of the 6th International Congress on Engineering and Food, Chiba, Japan, Vol. 2, Blackie, Glasgow). Decaffeination was measured as a function of CO₂ flow rate, temperature and pressure. The rate of decaffeination increased with both temperature and pressure. The mathematical model describes the external and intraparticle diffusion resistances and the distribution of caffeine between water and CO₂. The partition coefficient for caffeine distributed between water and supercritical CO₂ depends on temperature and pressure; soaking the raw beans in water prior to decaffeination enhances the rate of decaffeination.

WO94/26125 published a new decaffeination system based on supercritical CO₂ under conventional conditions, but with the addition of a liquid water stream on top of the moistened green coffee layer in the high pressure vessel. The amount of water used is 1-4 kg per kg green coffee (dry matter); if applied as continuous flow in the water saturated CO₂ atmosphere, it improves the mass transfer of caffeine from the bean surface to the fluid and reduces the decaffeination, time by about 50%. If applied in pulses of about 9 minutes, with one to four pulses per hour, then a high percentage of chlorogenic acid (considered undesirable) is removed and can be absorbed in the CO₂ washing column in water.

US Patent 4 411 923 proposed the replacement of activated carbon by ion-exchangers in order to adsorb the caffeine out of the supercritical CO₂-stream. It was found that strong acidic cation exchangers are considerably more selective than activated carbon, that they can be regenerated with aqueous salt solutions or mineral acids and that they are pressure resistant. To recover the caffeine, the aqueous regenerant solutions are either concentrated until the caffeine crystallizes out or they are subjected to a liquid-liquid extraction, for example with methylene chloride.

In Tokunaga, Y., Fujii, T. & Nakamura, K. (1997) Biosci. Biotech. Biochem., 61, 1024-1026, laboratory tests have been published in which activated carbon was replaced by a zeolite membrane to separate the caffeine from the supercritical fluid. The zeolite was coated as a thin layer on the surface of a tubular alumina membrane by hydrothermal synthesis. The system has turned out to have high thermal and pressure resistance and a good caffeine separation performance. The caffeine recovery was not reported.

An application of the supercritical CO₂ decaffeination process to roasted coffee (or tea leaves) has been described by EP 0151202. In the first step, the aroma components are extracted by dry supercritical CO₂ and in the second step, the wetted roast coffee is decaffeinated under the usual conditions with CO₂. Thereafter, the water soluble components are extracted from the decaffeinated roast coffee. The coffee extract is mixed with the aroma-containing supercritical dry CO₂, out of which the aroma components condense into the liquid after decompression; the extract can then be freeze dried.

### Phytochemical- or Polyphenol-enriched Extract (or Fraction)

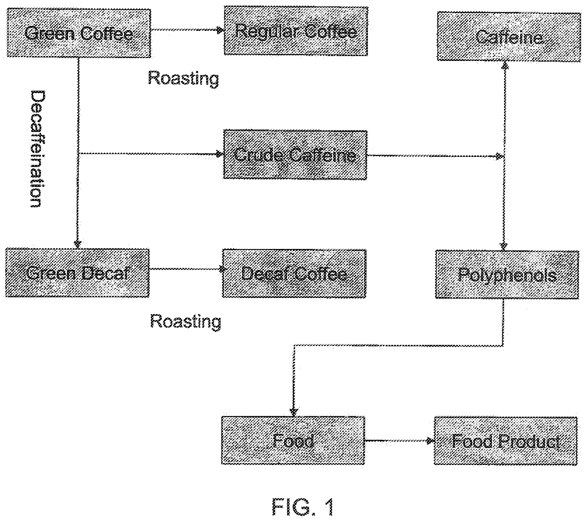

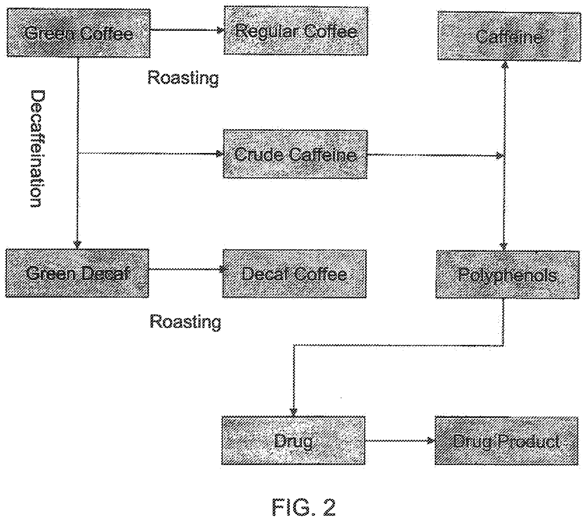

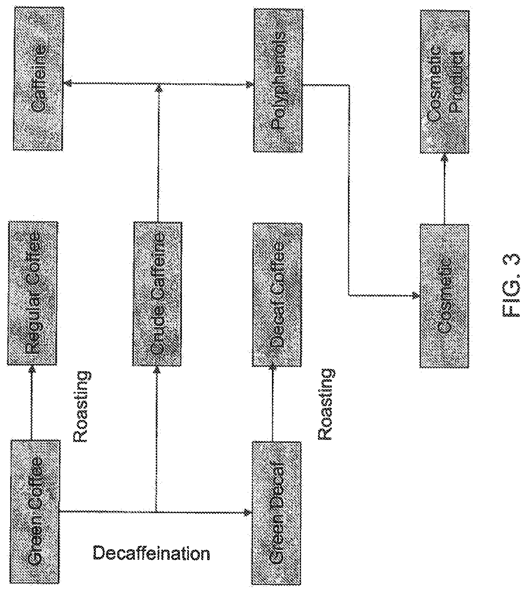

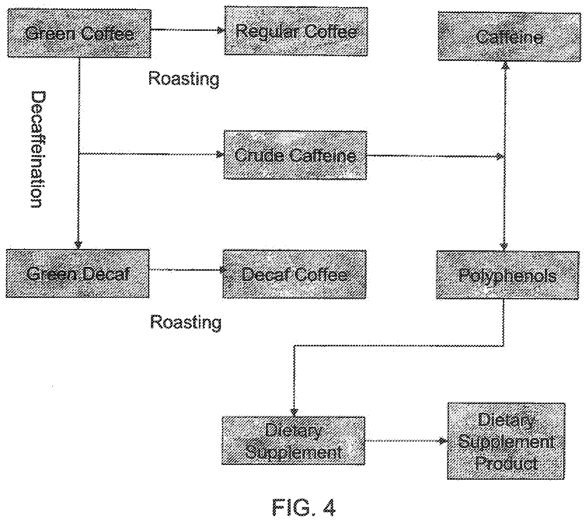

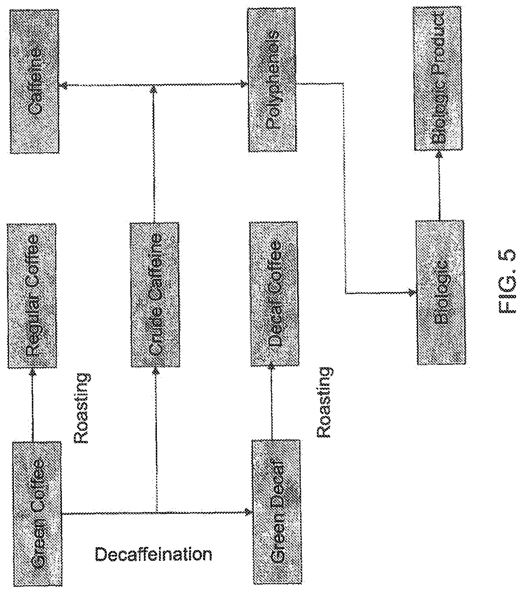

In one embodiment, referring to FIG. 1 to 5, green coffee is extracted by supercritical carbon dioxide decaffeination to produce crude caffeine. The crude caffeine, or its vacuum-dried product resuspended in water, is further processed to reduce caffeine and enrich polyphenols by recovering the retentate of a filtration process of a water suspension of crude caffeine as a phytochemical- or polyphenol-enriched extract (or fraction). A food (FIG. 1), drug (FIG. 2), cosmetic (FIG. 3), dietary supplement (FIG. 4), or biologic (FIG. 5) is supplemented with, or a component of a food (FIG, 1), drug (FIG. 2), cosmetic (FIG. 3), dietary supplement (FIG. 4), or biologic (FIG, 5) is supplemented with, the phytochemical- or polyphenol-enriched extract (or fraction) to produce a food product (FIG. 1), drug product (FIG. 2), cosmetic product (FIG. 3), dietary supplement product (FIG. 4), or biologic product (FIG. 5). In FIG. 1 to 5, the phytochemical- or polyphenol-enriched extract (or fraction) functions as either an active ingredient or, alternatively, an additive or excipient or inert (non-therapeutic) ingredient. As an example of the latter case, the phytochemical- or polyphenol-enriched extract (or fraction) functions as an antioxidant to improve stability or shelf-life of the food product (FIG. 1), drug product (FIG. 2), cosmetic product (FIG. 3), dietary supplement product (FIG. 4), or biologic product (FIG. 5). As an example of the former case, the phytochemical- or polyphenol-enriched extract (or fraction) functions as an active ingredient of the product to stimulate glucose uptake, inhibit COX-2, or act as a neuroprotectant, or, additionally, to protect against oxygen radicals that damage tissue or the skin.

We devised an enrichment method to isolate antioxidants from crude caffeine. In one embodiment, crude caffeine is a byproduct of the supercritical carbon dioxide (scCO₂) decaffeination process. In one example, green coffee beans are soaked in water until the moisture content is 50%. Caffeine is removed in an extractor by liquid carbon dioxide at high temperature (90-100°C) and high pressure (300 atm). The liquid carbon dioxide is re-circulated between the extractor and a scrubber, where caffeine is removed from the liquid carbon dioxide with water. The resulting caffeine-rich aqueous solution is then concentrated by reverse osmosis and vacuum-dried.

In one embodiment, the proximate composition of crude caffeine is obtained. In one example, the primary components are found to be caffeine (95.95%), moisture (1.10%), and fat (1.04%); amounts of ash, fiber, protein, and sugar are negligible.

Phenolic compounds contribute about 10% of the mass of green coffee (Chu, Y. F. et al., Journal of Agricultural and Food Chemistry 2009, 57, 9801-9808). In one embodiment, the presence of phenolics in the crude caffeine is determined using, in one instance, the Folin-Ciocalteu assay. In one example, the level of total phenolics is found to be 10 mg CE/g, or approximately 1% of crude caffeine by mass.

In one embodiment, the antioxidant activity of the hydrophilic and lipophilic fractions of crude caffeine is assessed. In one example, the ORAC_{hydro} is found to be 145 µmol TE/g and the ORACₗᵢₚₒ is found to be 66 µmol TE/g, whereas pure caffeine is found to be associated with an extremely low ORAC_{hydro} (6 µmol TE/g) and ORACₗᵢₚₒ (0 µmol TE/g).

In one embodiment, a biological correlate of the crude caffeine's *in vitro* antioxidant activity is evaluated. In one example, primary cortical neuron cells are pretreated with crude caffeine before H₂O₂-induced oxidative stress. In one control, H₂O₂ diminishes cell survival in a dose-dependent manner. In this example, pure caffeine is found to be not toxic to primary neuron cells, but does not protect against H₂O₂-induced cell death. Similarly, in this example, crude caffeine is found to be not toxic to primary neuron cells, but does not protect against H₂O₂-induced cell death, even though ORAC values of crude caffeine are much higher than those of pure caffeine.

In one embodiment, the anti-hyperglycemic effects of crude caffeine on glucose uptake in human skeletal muscle cells and adipocytes are determined, using insulin as a positive control. In one example, 100 nM insulin stimulates a 2.06-fold increase in glucose uptake in human skeletal muscle cells, and although less effective than insulin, 0.01 mg/mL crude caffeine increases glucose uptake significantly by 1.45 fold. In contrast, in this example, 0.01 mg/mL pure caffeine produces no significant effect. In one example, crude caffeine stimulates glucose uptake more effectively than insulin in human adipocytes: 0.01 mg/mL crude caffeine induces a 2.20-fold increase in glucose uptake, whereas 100 nM insulin induces a 1.6-fold increase. In this example, pure caffeine (0.01 mg/mL) does not exert a significant effect on glucose uptake.

In one embodiment, the anti-inflammatory activity of crude caffeine is evaluated by determining its effect on the inflammatory enzyme COX-2, using aspirin as a positive control. In one example, crude caffeine inhibits COX-2 activity; the IC₅₀ value is 20 µg/mL, which is 9.5 times lower than that of aspirin (IC₅₀, 190 µg/mL), indicating that crude caffeine is a more potent COX-2 inhibitor than aspirin. In contrast, in this example, the activity of regular coffee (IC₅₀, 2010 µg/mL) is about 10 times lower than that of aspirin. In this example, pure caffeine does not inhibit COX-2 activity.

In one embodiment, the crude caffeine is further processed to reduce caffeine and enrich polyphenols. The crude caffeine obtained from supercritical carbon dioxide decaffeination, or its vacuum-dried product resuspended in water, is suspended to form a suspension, and the water-insoluble particles (WIP) of this suspension are collected by filtration. In one example, this method reduces the caffeine content below 5%. In one example, the WIP fraction contains only 1.6% caffeine, about 60 times less than crude caffeine. In one example, the presence of phenolics in the WIP fraction is determined using, in one instance, the Folin-Ciocalteu assay. In one example, the level of total phenolics is found to be 313 mg CE/g, or approximately 30% of the WIP fraction by mass. In one example, the ORAC_{hydro} and ORACₗᵢₚₒ values of the WIP fraction are found to be 1321 µmol TE/g and 332 µmol TE/g, respectively, about 9 and 5 times higher, respectively, than those of crude caffeine. In one example, the antioxidant activity of WIP is evaluated in mouse primary neuron cells. In one example, WIP show no toxicity and significantly ameliorate cell death in H₂O₂-initiated oxidative stress in a dose-dependent manner. In one example, WIP (1000 µg/mL) increase survival of mouse primary neuron cells treated with H₂O₂ (250-1000 µM) by about 3.5-fold. In one embodiment, the ratio of total phenolics to caffeine is about 20, 10-30 or 40, or greater than 10.

In one embodiment, antioxidant activity-guided fractionation and LC-MS analysis of WIP is performed. In one example, WIP are separated into multiple fractions by flash chromatography on a C-18 column, and the ORAC_{hydro} of each fraction is determined. In one example, fraction(s) that demonstrate the highest antioxidant capacities are submitted to LC-MS analysis.

In one embodiment, seven chemicals are identified in the fractions with the highest antioxidant capacities: caffeic acid, coumaric acid, vanillic acid, quercetin, catechin, abscisic acid glucose ester, and threonine. Among them, caffeic acid, coumaric acid, vanillic acid, quercetin, and catechin are well-known phenolic antioxidants. Abscisic acid is a plant hormone but when present as a glucose ester conjugate is inactive; and threonine is an amino acid.

In one embodiment the synergistic effects of crude caffeine and WIP are determined when combined with other biological antioxidants. In one example, an ORAC assay is adapted for the linoleate microemulsion system (Sim, W. L. S. et al., Journal of Agricultural and Food Chemistry 2009, 57, 3409-3414) to determine the antioxidant activity of α-tocopherol (a form of vitamin E) combined with crude caffeine, WIP, ascorbic acid, catechin, chlorogenic acid, WIP + catechin, or WIP + chlorogenic acid. In this example, data are expressed as relative ORAC: a relative ORAC of 100% means that the antioxidant activity of the mixture equals the sum of each component's antioxidant activity (additive effect), whereas a relative ORAC greater than 100% means that the antioxidant activity of the mixture is greater than the sum of each component's antioxidant activity (synergistic effect). In one example, crude caffeine and ascorbic acid produce an additive antioxidant effect in combination with α-tocopherol. In contrast, in this example, WIP, catechin, and chlorogenic acid produce a synergistic effect in combination with α-tocopherol.

### Extractions

Extraction, as the term is used pharmaceutically, involves the separation of medicinally active portions of plant or animal tissues from the inactive or inert components by using selective solvents in different extraction procedures. The products obtained from plants are relatively impure concentrates intended mostly for oral or external use. The resultant preparations are popularly called galenicals.

### Drugs and Biologics Manufacturing

Drug substances most frequently are administered orally by means of solid dosage forms such as tablets and capsules, although powders can also be administered as the simplest dosage form. Large-scale production methods used for the preparation of tablets and capsules usually require the presence of other materials in addition to the active ingredients. Additives also may be included in the formulations to facilitate handling, enhance the physical appearance, improve stability, and aid in the delivery of the drug to the bloodstream after administration.

Tablets may be defined as solid pharmaceutical dosage forms containing drug substances with or without suitable diluents and have been traditionally prepared by either compression, or molding methods. Recently, punching of laminated sheets, electronic deposition methods, and three-dimensional printing methods have been used to make tablets.

Compressed tablets are formed by compression and in their simplest form, contain no special coating. They are made from powdered, crystalline, or granular materials, alone or in combination with binders, disintegrants, controlled-release polymers, lubricants, diluents, and in many cases colorants. The vast majority of tablets commercialized today are compressed tablets, either in an uncoated or coated state.

The basic mechanical unit in all tablet-compression equipment includes a lower punch that fits into a die from the bottom and an upper punch, with a head of the same shape and dimensions, which enters the die cavity from the top after the tableting material fills the die cavity. The tablet is formed by pressure applied on the punches and subsequently is ejected from the die. There are three general methods typically used for commercial tablet preparation: the wet-granulation method, the dry-granulation method, and direct compression.

In addition to the active or therapeutic ingredient, tablets contain a number of inert materials. The latter are known as additives or excipients. They may be classified according to the part they play in the finished tablet. The first group contains those that help to impart satisfactory processing and compression characteristics to the formulation. These include diluents (*e.g*., dicalcium phosphate, Calcium sulfate, lactose, cellulose, kaolin, mannitol, sodium chloride, dry starch, and powdered sugar), binders (*e.g*., starch, gelatin, sugars, gums, cellulosics, and polyvinylpyrrolidone), glidants (*e.g*., colloidal silicon dioxide), and lubricants (*e.g*., talc, magnesium stearate, calcium stearate, stearic acid, glyceryl behanate, hydrogenated vegetable oils, and polyethylene glycol). The second group of added substances helps to give additional desirable physical characteristics to the finished tablet. Included in this group are disintegrants (*e.g*., starches, clays, celluloses, algins, gums, and cross-linked polymers), surfactants, colors, and, in the case of chewable tablets, flavors, and sweetening agents, and in the case of controlled-release tablets, polymers or hydrophobic materials, such as waxes or other solubility-retarding materials. In some cases, antioxidants or other materials can be added to improve stability and shelf-life.

The most widely used and most general method of table preparation is the wet-granulation method. The steps in this method constitute weighing, mixing, granulation, wet screening, drying, dry screening, lubrication, and compression. One wet granulation formulation for CT Ascorbic Acid USP, 50 mg, is:

| Ingredients | In each |
|---|---|
| Ascorbic acid USP | 55 mg |
| Lactose | 21 mg |
| Starch | 13 mg |
| Ethylcellulose N 100 | 16 mg |
| Starch | 7 mg |
| Talc | 6.5 mg |
| Calcium stearate | 1 mg |

Granulate the first three ingredients with ethylcellulose (5%) dissolved in anhydrous ethyl alcohol, adding additional anhydrous alcohol to obtain wet granules. Wet-screen through a stainless steel screen and dry at room temperature. Dry-screen through a stainless steel screen and incorporate the remaining three ingredients. Mix thoroughly and compress. Use a flat, beveled 1/4-inch punch.

Dry granulation includes weighing, mixing, slugging, dry screening, lubrication, and compression. One dry granulation formulation for CT Vitamin B Complex is:

| Ingredients | In each |
|---|---|
| Thiamine mononitrate | 0.733 mg |
| Riboflavin | 0.733 mg |
| Pyridoxine hydrochloride | 0.333 mg |
| Calcium pantothenate | 0,4 mg |
| Nicotinamide | 5 mg |
| Lactose | 75.2 mg |
| Starch | 21.9 mg |
| Tale | 20 mg |
| Stearic acid | 0.701 mg |

Mix all the ingredients together. Compress into slugs. Grind and screen to mesh granules. Recompress into tablets, using a 1/4 -inch concave punch.

Direct compression entails compressing tablets directly from powdered material. It is cheaper but not always compatible with raw materials. One direct compression formulation for CT Ascorbic Acid USP, 250 mag, is:

| Ingredients | In each |
|---|---|
| Ascorbic acid USP | 255 mg |
| Microcrystalline cellulose | 159 mg |
| Stearic acid | 9 mg |
| Colloidal silica | 2 mg |

Blend all ingredients in a suitable blender. Compress, using a 1/4-inch standard concave punch.

Capsules are solid dosage forms in which the drug substance is enclosed in either a hard or soft, soluble shell of a suitable form of gelatin. Compared with tablets, powders for filling into hard gelatin capsules require a minimum of formulation efforts. The powders usually contain diluents such as lactose, mannitol, calcium carbonate, or magnesium carbonate. Lubricants such as the stearates also are used frequently. The gelatin for soft shell capsules is plasticized typically by the addition of glycerin, sorbitol, or a similar polyol.

Medicated chewing gum can be manufactured by a variety of mixing processes that incorporate several components into a sheet of product, whereby the units are stamped or cut from the rolled out sheet. A typical formulation for a medicated chewing gum is:

| Component | Concentration (% w/w) |
|---|---|
| Drug | 0-40 |
| Gum Base | 20-45 |
| Sweeteners | 30-60 |
| Softeners | 0-10 |
| Flavors | 1-5 |
| Colors | 0-1 |

Medicated chewing gums can be made by compression and other processes, but the predominant method in use today is mixing, rolling, and stamping of the finished units.

New drug development can proceed along varied pathways for different compounds, but a development paradigm has been articulated that has long served well as a general model. In outline form, the paradigm portrays new drug discovery and development as proceeding in a sequence of (possibly overlapping) phases. Discovery programs result in the synthesis of compounds that are tested in preclinical tests called assays and animal models. Clinical (human) testing typically proceeds through three successive phases. In phase I, a small number of usually healthy volunteers are tested to establish safe dosages and to gather information on the absorption, distribution, metabolic effects, excretion, and toxicity of the compound. To conduct clinical testing in the United States, a manufacturer must first file an investigational new drug application (IND) with the FDA. Phase II trials are conducted with subjects who have the targeted disease or condition and are designed to obtain evidence on safety and preliminary data on efficacy. The number of subjects tested in this phase is larger than in phase I and may number in the hundreds. The final pre-approval clinical testing phase, phase III, typically consists of a number of large-scale (often multi-center) trials that are designed to firmly establish efficacy and to uncover side-effects that occur infrequently. The number of subjects in phase III trials for a compound can total in the thousands. Once drug developers believe that they have enough evidence of safety and efficacy, they will compile the results of their testing in an application to regulatory authorities for marketing approval. In the United States, manufacturers submit a new drug application (NDA) or a biological license application (BLA) to the FDA for review and approval.

### Diabetes Mellitus

Diabetes mellitus (DM) consists of a group of syndromes characterized by hyperglycemia; altered metabolism of lipids, carbohydrates, and proteins; and an increased risk of complications from vascular disease. Most patients can be classified clinically as having either type 1 or type 2. DM or carbohydrate intolerance also is associated with certain other conditions or syndromes. Criteria for the diagnosis of DM have been proposed by several medical organizations. The American Diabetes Association (ADA) criteria include a random plasma glucose concentration of greater than 200 mg/dl, a fasting plasma glucose concentration of greater than 126 mg/dl , or a plasma glucose concentration of greater than 200 mg/dl 2 hours after the ingestion of an oral glucose load. Diabetes Care 2003, 26, s5-20.

Virtually all forms of DM are caused by a decrease in the circulating concentration of insulin (insulin deficiency) and a decrease in the response of peripheral tissues to insulin (insulin resistance). These abnormalities lead to alterations in the metabolism of carbohydrates, lipids, ketones, and amino acids; the central feature of the syndrome is hyperglycemia. Insulin lowers the concentration of glucose in blood by inhibiting hepatic glucose production and by stimulating the uptake and metabolism of glucose by muscle and adipose tissue.

Near-normoglycemia can be attained in patients with insulin and oral hypoglycemic agents. The goal is to achieve a fasting blood glucose concentration of between 90 and 120 mg/dl and a 2-hour postprandial value below 150 mg/dl . In less disciplined patients, or in those with defective responses of counter-regulatory hormones, it may be necessary to accept higher fasting (*e.g*., 140 mg/dl) and 2-hour postprandial (*e.g*., 200 to 250 mg/dl) blood glucose concentrations.

Crude caffeine stimulates glucose uptake in human fat and muscle cells. A suitable anti-diabetic agent should have actions similar to those of insulin, or it should bypass the defects in insulin action characterized by insulin resistance. We evaluated the insulinomimetic activity of crude caffeine working with two primary human cell cultures of insulinsensitive tissues: myotubes and adipocytes. FDA clearance may require animal testing to assess how effective a crude caffeine or fraction thereof is at lowering hyperglycemia, *e.g*., in an insulin-deficient mouse model of diabetes, such as C57BL/6 and *ob* and *db* mutants. It may require further assays to evaluate the insulinomimetic activity of a crude caffeine or fraction thereof, *e.g*., working with organ cultures or primary cell cultures or cell culture lines of insulinsensitive tissues, such as L6E9 myotubes and 3T3-L1 adipocytes. The data argue for clinical (human) testing to establish efficacy and optimum dosage of these extracts for stimulating glucose uptake and treating diabetes mellitus in human subjects having or at risk for developing diabetes mellitus.

### Inflammation

The inflammatory process is the response to an injurious stimulus. Prostaglandins participate in the pathogenesis of inflammation. Aspirin and NSAIDS (non-steroidal anti-inflammatory drugs) inhibit the biosynthesis of prostaglandins. The first enzyme in the prostaglandin synthetic pathway is cyclooxygenase or COX. This enzyme converts arachidonic acid to unstable intermediates and leads to the production of thromboxane A2 and a variety of prostaglandins. Therapeutic doses of aspirin and NSAIDS inhibit the COX enzymes and prostaglandin production.

There are two forms of cyclooxygenase, cyclooxygenase-1 (COX-1) and cyclooxygenase-2 (COX-2). COX-1 is found in most normal cells and tissues, while cytokines and inflammatory mediators that accompany inflammation induce COX-2 production. COX-1, but not COX-2, is expressed in gastric epithelial cells. Inhibition of COX-1 at this site is thought to account largely for the gastric adverse events that complicate therapy with NSAIDS, thus providing the rationale for the development of NSAIDS specific for inhibition of COX-2.

Most NSAIDS inhibit both COX-1 and COX-2 with little selectivity. The hypothesis that the anti-inflammatory effects of NSAIDS would be accompanied by a lower ulcerogenic potential propelled efforts to design drugs with greater selectivity for COX-2 versus COX-1. These efforts led to the approval and marketing of *e.g.,* celecoxib as selective COX-2 inhibitors.

Aspirin covalently modifies COX-1 and COX-2, irreversibly inhibiting cyclooxygenase activity. In contrast, the vast majority of NSAIDS act as reversible, competitive inhibitors of cyclooxygenase activity. The selective COX-2 inhibitors have been shown to be less prone than equally efficacious doses of NSAIDS to induce gastric ulcers, and this has provided the basis of FDA approval of, *e.g*., celecoxib.

NSAIDS find their chief clinical application as anti-inflammatory agents in the treatment of musculoskeletal disorders, such as arthritis. The word arthritis actually means joint inflammation and extends to fibromyalgia, gout, and lupus. Of the two common forms, osteoarthritis is more prevalent than rheumatoid arthritis.

Osteoarthritis is a disease characterized by degeneration of cartilage and its underlying bone within a joint and involves inflammation. The American College of Rheumatology (ACR) has published clinical classification guidelines for osteoarthritis of the hand (Altman, R. et al., Arthritis Rheum 1990, 33, 1601-10), hip (Altman, R. et al., Arthritis Rheum 1991, 34, 505-14), and knee (Altman, R, et al., Arthritis Rheum 1986, 29, 1039-49). An algorithm for the management of osteoarthritis of the knee is provided in Hochberg MC et al., Arthritis Rheum 1995, 38, 1541-6, and an algorithm for the management of osteoarthritis of the hip is provided in Hochberg MC et al., Arthritis Rheum 1995, 38, 1535-40.

Rheumatoid arthritis is a systemic inflammatory disease which manifests itself in multiple joints of the body, leading to inflamed synovium (lining of the joint) and erosion of cartilage and bone. The 1987 American College of Rheumatology criteria are used in the clinical diagnosis of rheumatoid arthritis, and to define rheumatoid arthritis in epidemiologic studies. Persons must meet four of seven ACR criteria; these criteria are based on clinical observation (*e.g*., number of joints affected), laboratory tests (*e.g.,* positive rheumatoid factor), and radiographic examination (*e.g.,* X-rays evidence of joint erosion). Arnett FC et al., Arthritis Rheum 1988, 31, 315-324.

Historically, pharmacologic treatment of RA has traditionally followed the pyramid approach. That is, treatment starts with corticosteroids/non-steroidal anti-inflammatory drugs, then progresses to disease-modifying anti-rheumatic drugs and finally to biologic response modifiers if persons are non-responsive to the previous drugs. Arthritis Rheum 2002, 46, 328-346.

Crude caffeine inhibits the inflammatory enzyme COX-2. We evaluated the COX-2 activity of crude caffeine measured by assessing oxygen consumption in reactions started by adding arachidonic acid to a mixture containing among other things heme together with recombinant human COX-2; and the test material. FDA clearance may require animal testing to assess how effective a crude caffeine or fraction thereof is at inhibiting COX-2, *e.g*., in models of arthritis, with the most widely used models being adjuvant-induced arthritis, collage-induced arthritis, antigen-induced arthritis, and streptococcal cell wall arthritis, and, more recently, transgenic models. It may require further *in vitro* assays to evaluate the COX-2 activity of a crude caffeine or fraction thereof, *e.g.,* measured by the human whole-blood assay developed by Patrignani, P. et al., J Pharmacol Exp Ther 1994, 271, 1705-12 and agreed by the International Consensus Meeting on the Mode of Action of COX-2 Inhibition in December 1997 as the *in vitro* assay that should be adopted as the standard method for assessing inhibition of COX-2 and COX-1. The data argue for clinical (human) testing to establish efficacy and optimum dosage of these extracts for inhibiting COX-2 and treating inflammation in human subjects having or at risk for developing inflammation.

### Dementia

Alzheimer's disease is the major cause for dementia in later life. Dementia is a broad term referring to a decline in cognitive function to the extent that it interferes with daily life and activities. The most widely accepted diagnostic criteria for probable Alzheimer's disease are those offered by the National Institute of Neurological and Communicative Disorders and Stroke and by the Alzheimer's Disease and Related Disorders Association (NINCDS-ADRDA; McKhann G. et al., Neurology 1984, 34, 939-44). These criteria include the presence of dementia established by clinical examination and confirmed by neuropsychological testing. The dementia is described as involving multiple, progressive cognitive deficits in older persons in the absence of disturbances of consciousness, presence of psychoactive substances, or any other medical, neurological, or psychiatric conditions that might in and of themselves account for these progressive deficits. The Diagnostic and Statistical Manual of Mental Disorders: 4th Edition of the American Psychiatric Association (DSM-IV, 1994) also outlines diagnostic criteria for dementia of the Alzheimer's type that are generally consistent with the NINCDS-ADRDA criteria. DSM-IV also provides diagnostic criteria for vascular dementia, as well as dementia due to other general medical conditions including HIV disease, head trauma, Parkinson's disease, Huntington's disease, Pick's disease, Creutzfeldt-Jakob disease, and other general medical conditions and etiologies. New causes and varieties of dementia continue to be elucidated and diagnostic criteria for the dementing disorders continue to be refined.

The National Institute on Aging (NIA) and Alzheimer's Association are leading an effort to update the diagnostic criteria for Alzheimer's disease. Among the most important advances in the Alzheimer's field since the publication of the 1984 diagnostic criteria are: (1) Alzheimer's-driven changes in the brain, as well as the accompanying cognitive deficits, develop slowly over many years with dementia representing the end stage of years of pathology accumulation; (2) Predictive genes in early onset Alzheimer's indicate that the initial events ultimately leading to both clinical symptoms and pathological brain changes begin with disordered beta amyloid metabolism; (3) The e4 allele of the APOE gene is well accepted as a major genetic risk factor for late onset Alzheimer's disease, which is defined as onset at 65 or older; and (4) Biomarkers for Alzheimer's have been developed and are being validated. These fall into several categories: (a) Biomarkers of beta amyloid pathology, including amyloid positron emission tomography (PET) imaging and levels of beta amyloid in cerebrospinal fluid (CSF); (b) Biomarkers of neuronal injury, including levels of CSF tau and phospho-tau; (c) Biomarkers of neuronal dysfunction, including decreased uptake of fluorodeoxyglucose (FDG) on PET scans; and (d) Biomarkers of neurodegeneration, including brain atrophy on structural magnetic resonance imaging (MRI) scans.

The NIA/Alzheimer's Association working groups are organized around the three stages of Alzheimer's disease that are commonly thought to exist today - pre-clinical Alzheimer's, mild cognitive impairment (MCI) due to Alzheimer's, and Alzheimer's dementia. The "pre-clinical" group is laying out a research agenda to identify methods of assessment that may help predict risk for developing the disease. Biomarkers and other clinical assessment tools to identify early cognitive decline are being investigated to establish the presence of Alzheimer's brain changes in people with no overt symptoms and to identify those who may eventually develop the disease. The "MCI" group is refining the MCI criteria, which will help to indicate cognitive change before dementia and better differentiate MCI from Alzheimer's. Research is underway to better understand the cognitive changes taking place, how they may relate to biomarkers, and which of these methods best indicate the likelihood of imminent progression to Alzheimer's dementia. The "Alzheimer's dementia" group is revising the existing criteria for diagnosing Alzheimer's to include possible biomarkers and other assessments that may aid in diagnosis. After the draft reports from the three workgroups are finalized, the next steps are publication in a peer-reviewed journal followed by systematic validation through incorporation of the criteria into clinical trials.

Declines in memory and cognitive abilities are actually a normal consequence of aging in humans. The new category of "age associated memory impairment" was proposed by a National Institute of Mental Health (NIMH) work group to describe older persons with objective memory declines relative to their younger years, but cognitive functioning that is normal relative to their age peers (Crook, T.H. et al., Developmental Neuropsychology, 1986, 2, 261-276). The group's recommendations contained explicit operational definitions and psychometric criteria to assist in identifying these persons. Other terms for this condition include "age consistent memory decline" and "age related cognitive decline." The DSM-IV (1994) has codified the diagnostic classification of age related cognitive decline.

A WIP fraction of crude caffeine, reduced in caffeine and enriched for polyphenols, besides concentrating antioxidants potentiates neuroprotectant activity. We evaluated the neuroprotectant activity of a WIP fraction of crude caffeine working with mouse primary neuron cells. FDA clearance may require animal testing to assess how effective a crude caffeine or fraction thereof is at protecting neurons, *e.g.,* in a mouse model of Alzheimer's disease, such as tau models, APP models, secretase models, ApoE models, and axonal transport models; or, *e.g.,* in an animal model of Parkinson's disease; or, *e.g*., in an animal model of Huntington's disease. It may require further assays to evaluate the neuroprotectant activity of a crude caffeine or fraction thereof working with organotypic brain slice cultures or neuronal tissue culture preparations. The data argue for clinical (human) testing to establish efficacy and optimum dosage of these extracts for neuroprotectant activity and treating dementia and age related cognitive decline in human subjects having or at risk for developing dementia and age related cognitive decline.

### Dosages

The dose of a drug required to produce a specified effect in 50% of the population is the median effective dose, abbreviated ED₅₀. In preclinical studies of drugs, the median lethal dose, as determined in experimental animals, is abbreviated as the LD₅₀. The ratio of the LD₅₀ to the ED₅₀ is an indication of the therapeutic index, which is a statement of how selective the drug is in producing the desired versus its adverse effects. Drugs that exhibit high therapeutic indices are preferred.

The dosage of the extracts is on an order that takes the ED₅₀., LD₅₀, and therapeutic index and the following into account. The total intake of dietary phenolics is ~ 1 g/d. The maximum plasma concentration is in the range of 0.1 to 10 µmol/L after the consumption of 10 to 100 mg of a single phenolic compound.

The food, drug, cosmetic, dietary supplement, or biologic product contains about 0.05% extract by weight, about 0.1% extract by weight, about 1% extract by weight, about 5% extract by weight, about 10% extract by weight, about 15% extract by weight, about 20% extract by weight, about 25% extract by weight, about 30% extract by weight, about 35% extract by weight, about 40% extract by weight, about 45% extract by weight, about 50% extract by weight, about 55% extract by weight, about 60% extract by weight, about 65% extract by weight, about 70% extract by weight, about 75% extract by weight, about 80% extract by weight, about 85% extract by weight, about 90% extract by weight, about 95% extract by weight, or about 99% extract by weight.

### Food Manufacturing

A food additive is defined in Section 201(s) of the FD&C Act as any substance the intended use of which results or may reasonably be expected to result, directly or indirectly, in its becoming a component or otherwise affecting the characteristic of any food (including any substance intended for use in producing, manufacturing, packing, processing, preparing, treating, packaging, transporting, or holding food; and including any source of radiation intended for any such use); if such substance is not GRAS or sanctioned prior to 1958 or otherwise excluded from the definition of food additives.

"GRAS" is an acronym for the phrase Generally Recognized As Safe. Under sections 201(s) and 409 of the FD&C Act, any substance that is intentionally added to food is a food additive, that is subject to premarket review and approval by FDA, unless the substance is generally recognized, among qualified experts, as having been adequately shown to be safe under the conditions of its intended use, or unless the use of the substance is otherwise excluded from the definition of a food additive.

### Cosmetics Manufacturing

Cosmetic ingredients are identified, under the FD&C Act, by the name established by the Commissioner for the purpose of cosmetic ingredient labeling or, in the absence of a name established by the Commissioner, the name adopted for that ingredient in the editions and supplements of the following compendia: (a) CTFA (Cosmetic, Toiletry and Fragrance Association, Inc.) Cosmetic Ingredient Dictionary; (b) United States Pharmacopeia; (c) National Formulary; (d) Food Chemical Codex; and (e) USAN and the USP Dictionary of Drug Names. If none lists a name for an ingredient, the name generally recognized by consumers, or the chemical or technical name or description, is then used.

### Dietary Supplements Manufacturing

The types of ingredients in dietary supplements characteristically include other food ingredients (*e.g.,* water and sugar), and technical additives or processing aids (*e.g.,* gelatin, starch, colors, stabilizers, preservatives, and flavors).

### Example 1

Chemicals. All reagents were purchased from Sigma-Aldrich (St. Louis, MO) unless otherwise specified. High performance liquid chromatography (HPLC)-grade water was purchased from EMD (Gibbstown, NJ), and 2,2'-azobis (2-amidinopropane) dihydrochloride (ABAP) was purchased from Wako Chemicals USA (Richmond, VA). Randomly methylated β-cyclodextrin (RMCD; Trappsol, pharmaceutical grade) was obtained from Cyclodextrin Technologies Development (High Springs, FL). Phosphate buffered saline (PBS), Williams' medium E (WME), and Hank's balanced salt solution (HBSS) were purchased from Gibco Life Technologies (Grand Island, NY). Fetal bovine serum (FBS) was obtained from Atlanta Biologicals (Lawrenceville, GA). Arachidonic acid and COX-2 enzyme were purchased from Cayman Chemical (Ann Arbor, MI). TNF-α was purchased from Biomyx Technology (San Diego, CA). Essential medium (11090081) and neural basal medium (21103049) were purchased from Invitrogen (Carlsbad, CA). Crude caffeine was obtained from Maximus Coffee Group (Houston, TX). The oxygen biosensor system (OBS), a special ruthenium dye-coated 96-well microplate, was purchased from BD Biosciences Discovery Labware (Bedford, MA).

Crude Caffeine. Crude caffeine was produced as a byproduct of the supercritical carbon dioxide (scCO₂) decaffeination process. Briefly, green coffee beans were soaked in water until the moisture content was 50%. Caffeine was removed in an extractor by liquid carbon dioxide at high temperature (90-100°C) and high pressure (300 atm). The liquid carbon dioxide was re-circulated between the extractor and a scrubber, where caffeine was removed from the liquid carbon dioxide with water. The resulting caffeine-rich aqueous solution was then concentrated by reverse osmosis and vacuum-dried. Proximate composition of the crude caffeine was analyzed by Silliker, Inc. (South Holland, IL).

Total phenolics. The total polyphenol content of the crude caffeine was determined according to the method of Singleton and Rossi (Singleton, V. L. and Rossi, J. A., Jr., Am. J. Enol. Vitic. 1965, 16, 144-158). One milliliter of chlorogenic acid standard or crude caffeine solution was mixed with 15 mL water and 1.0 mL Folin-Ciocalteu reagent, and then incubated at room temperature for 10 min. After adding 20% sodium carbonate (3.0 mL) and incubating at 40°C for 20 min, absorbance was measured at 755 nm with an Agilent 8453 UV-visible spectrophotometer (Waldbronn, Germany). The total polyphenol content was expressed in milligrams of chlorogenic acid equivalents per gram of crude caffeine (CE/g).

Oxygen radical absorbance capacity. To determine antioxidant values of the hydrophilic fraction (ORAC_{hydro)}, 5 g crude caffeine was extracted with 20 mL acetone/water (50:50 v/v) on an orbital shaker at room temperature for 1 h. The mixtures were centrifuged at 1972×g in a Rotanta 460R centrifuge (GMI, Ramsey, MN). The ORAC_{hydro} values of the supernatants were determined with a method adapted from Ou (Ou, B. et al., Journal of Agricultural and Food Chemistry 2001, 49, 4619-4626) on a FL600 plate fluorescence reader (Bio-Tek Instruments, Inc., Winooski, VT) controlled by KC4 3.0 software. The excitation wavelength was set at 485 (± 20) nm and the emission wavelength set at 530 (± 25) nm. To determine antioxidant values of the lipophilic fraction (ORACₗᵢₚₒ), 5 g crude caffeine was extracted twice with 10 mL hexane/dichloromethane (50:50 v/v). ORAC values of the combined organic phase were determined according to a previously published method (Wu, X. et al., Journal of Agricultural and Food Chemistry 2004, 52, 4026-4037; Huang, D. et al., Journal of Agricultural and Food Chemistry 2002, 50, 1815-1821).

Primary cortical neuronal culture. Primary neuronal cell cultures were generated from postnatal day 0 C57BL/6 mice. The cortex was removed in Hanks' balanced salt solution and dissociated by incubating in papain at 37°C for 30 min. Dissociated cells were seeded onto poly-L-lysine-coated plastic plates (Corning, Coming, NY) in minimal essential medium and incubated at 37°C with 5% CO₂ for 4 h. The medium was then changed to neural basal medium supplemented with B-27 and L-glutamine. To reduce proliferation of glial cells, 1 µM 5-fluoro-2'-deoxyuridine (FdU) was also added to the medium. After 4 days, the medium was replaced with neural basal medium without FdU, and cells were allowed to grow for an additional 4 days. All experiments were performed on cells incubated *in vitro* for 8 days. Immunocytochemistry indicated that more than 90% of the cultured cells were neurons.

Cell survival. Cell survival was quantified using the 3-(4,5-dimethylthiazol-2-yl)-2,5-diphenyltetrazolium bromide (MTT) assay, which measures mitochondrial succinate dehydrogenase conversion of MTT to purple formazan (Mosmann, T., Journal of Immunological Methods 1983, 65, 55-63; Cheng, I. H. et al., Nature Medicine 2004, 10, 1190-1192). Cells were seeded into 96-well plates (10⁵ cells/well) and grown for 24 h before experiments. The cells were pre-treated 0, 250, 500, or 1000 µg/mL crude or pure caffeine for 2 h. The medium was then removed, and cells were washed with fresh medium. Cells were then treated with hydrogen peroxide (H₂O₂, 0-1000 µM) for 30 min at 37°C with 5% CO₂. The cells were then incubated in medium containing MTT (0.5 mg/mL; Genemark, Taiwan) at 37°C for 4 h. After removing the medium, 100 µL of lysis buffer containing 10% SDS and 20 mM HCl was added to each well to dissolve the formazan crystals, and absorbance (570 nm) of each well were measured on a FL600 plate reader (Bio-Tek Instruments, Inc., Winooski, VT) controlled by KC4 3.0 software. Percent cell survival was calculated as (OD_{570 treated}/OD_{570 untreated})×100%.

Glucose uptake. The *in vitro* effects of crude caffeine on glucose uptake were assessed with human adipocytes and skeletal muscle cells (Zen-Bio, Research Triangle Park, NC). Briefly, primary human subcutaneous adipocytes or primary human myoblasts were differentiated in 96-well microplates. The resulting adipocytes (2 weeks post-differentiation) or skeletal muscle cells (10 days post-differentiation) were treated with crude caffeine (0.001-0.5 mg/mL, final concentration) in the presence of ³H-2-deoxyglucose; cells treated with insulin (100 nM) were used as the positive control, and untreated cells as the negative control. All samples, including the positive and negative controls, were tested in triplicate. After a 2-h incubation at 37°C and 5% CO₂, the cells were washed with PBS and lysed. The cell lysate was mixed with scintillation fluid and radioactivity of each well was measured as counts per minute (CPM).

COX-2 inhibition. Oxygen consumption was measured in the reaction chamber of an Oxytherm (Hansatech Instrumental, Norfolk, England) at 37°C. The reaction mixture consisting of 0.5 mL Tris buffer (0.1 M, pH 8.0), 5 µL heme [100 µM in dimethyl sulfoxide (DMSO)], and 10 µL of COX-2 enzyme was incubated for 1 min. Five microliters of each sample (in DMSO or ethanol) was added and the mixture was incubated for an additional 1 min. The assay was initiated by adding 5 µL arachidonic acid, and the oxygen concentration was monitored. The initial oxygen consumption rate was obtained from the kinetic curve. COX-2 inhibition was expressed as the inhibitor concentration at which the initial oxygen consumption rate decreased by 50% (IC₅₀).

Synergistic effects of bioactive compounds combined with alpha-tocopherol. In a 15-mL test tube, methyl linoleate (1.2 g), Tween-20 (2.9 g), *n*-butanol (1.5 g), and potassium phosphate buffer (4.4 g) were vortexed until the resulting solution was clear. An OBS microplate was prepared with duplicate wells of Trolox solution standards (20 µL; 500, 250, 125, 62.5, or 31.25 µM) and triplicate wells of samples diluted in phosphate buffer (20 µL). The perimeter wells were not used for kinetic studies to avoid potential edge effects. The oxidation substrate, methyl linoleate microemulsion (200 µL), was then added. The plate was incubated at 37°C for 10 min before adding 20 µL 2,2'-azobis(2-amidinopropane) dihydrochloride (AAPH, 0.20 g/mL in phosphate buffer) to each well; the final volume in each well was 240 µL. A control well containing 200 µL of microemulsion and 40 µL of phosphate buffer was used to normalize the fluorescence reading. The reaction kinetics were monitored at 37°C for 2 h, with readings every 2 min, by a Synergy HT microplate fluorescent reader (Biotek Instruments Inc.). The microplate reader was fitted with an excitation filter at 485 nm, an emission filter of 590 nm, and an oxygen sensor biosystem, and the plate was shaken for 20 s at low intensity before each reading to ensure sufficient mixing. After normalization, the fluorescence data were converted to the oxygen concentration at each point of the reaction. The ORAC values of samples were determined as previously described (Sim, W. L. S. et al., Journal of Agricultural and Food Chemistry 2009, 57, 3409-3414). Synergistic effects of the samples were presented as relative ORAC values, calculated as follows: 100% × [1+ (ORAC of the mixture - sum of the ORAC of each component)/ORAC of the mixture] based on a previous method (Sim, W. L. S. et al., Journal of Agricultural and Food Chemistry 2009, 57, 3409-3414).

Activity-guided fractionation and structure elucidation of crude caffeine compounds by liquid chromatography with tandem mass spectrometry (LC-MS/MS). The crude caffeine obtained from supercritical carbon dioxide decaffeination was further processed to reduce caffeine and enrich polyphenols. Since the crude caffeine was vacuum-dried, it was resuspended in water. 1 g crude caffeine was stirred for 15 min with 70 mL water at room temperature to form a suspension. The water-insoluble particles (WIP) of this suspension were collected by filtering through a 0.45-µm ZapCap-CR nylon filter (Whatman Inc., Piscataway, NJ) and vacuum-dried at room temperature. The overall mass yield was 1.1%. Proximate composition, ORAC, and total phenolic content of the dried WIP were analyzed as previously described.

Fractionation of 30 mg WIP was then carried out with a Yamazen Flash chromatography system AI-580 (San Bruno, CA), consisting of a gradient pump, UV detector set at 254 nm, fraction collector (FR340), and 16 × 60 mm Luknova C18 column (Mansfield, MA). The sample was eluted from the column with water and methanol; the water/methanol mixture varied from 9:1 to 15:85 over a period of 17 min, at a flow rate of 10 mL/min. Seventeen fractions (10 mL/fraction) were collected and vacuum-dried, and the ORAC values were determined.

Fractions with high ORAC values were further analyzed by LC-MS with a Shimadzu HPLC system (pump: LC-20AT; autosampler: SIL-HTC; UV detector: SPD-20A; Columbia, MD), equipped with a MAC-MOD HydroBond™ PS-C18 column (50 × 2.1 mm, 3 µm; MAC-MOD Analytical, Chadds Ford, PA). Mobile phase A consisted of water with 0.4% formic acid, and mobile phase B was acetonitrile. Samples (10 µL) were separated by HPLC at room temperature at a flow rate of 0.6 mL/min. The percentage of mobile phase B increased from 0% to 70% over the first 3 min, increased to 95% over the next 0.5 min, increased to 98% over the next 0.5 min, and was then maintained at 98% for 1.5 min. The percentage of mobile phase B was then decreased to 0% in 0.1 min and maintained at 0% for 5 min for column re-equilibration.

Detection was performed by an AB/MDS SCIEX 4000 Q-trap detector (Foster City, CA) with the ESI ionization source in negative mode, ion spray voltage of 4500 V, and temperature at 550°C. Multiple reaction monitoring (MRM) was used for chemical detection. Mass spectrometry differentiates chemicals with the same molecular weight by splitting each molecular structure into a unique pattern of fragments (chemical fingerprint). With the MRM technique, chemical structures of interest can be elucidated by the simultaneous detection of molecular ions and fragment ions.

Statistical analysis. Data were analyzed by one-way analysis of variance (ANOVA) followed by Tukey's post hoc comparison test using PASW Statistics 18 (Chicago, IL). Results are expressed as mean ± standard deviation (SD). P < 0.05 was considered statistically significant.

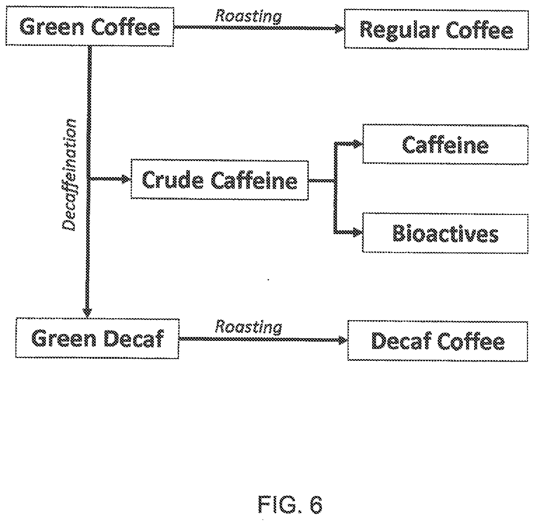

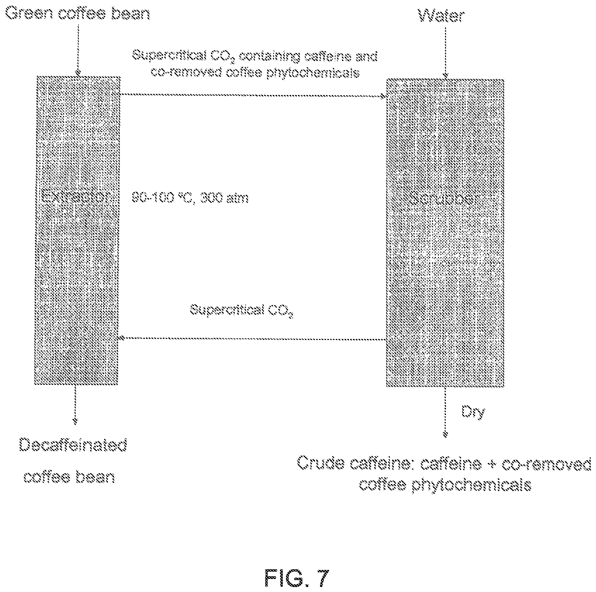

Results for proximate composition of crude caffeine. During coffee production, bioactive compounds turn up in the crude caffeine (FIG. 6). Coffee was decaffeinated by supercritical carbon dioxide (scCO₂) extraction (FIG. 7). The crude caffeine produced by scCO₂ decaffeination was evaluated.

Table 1 shows the proximate composition of crude caffeine. The primary components were caffeine (95.95%), moisture (1.10%), and fat (1.04%); amounts of ash, fiber, protein, and sugar were negligible.

We determined the presence of phenolics in the crude caffeine using the Folin-Ciocalteu assay, and found that the level of total phenolics was 10 mg CE/g (Table 2), or approximately 1 % of crude caffeine by mass.

Results for antioxidant activity of crude caffeine. The antioxidant activity of the hydrophilic and lipophilic fractions of crude caffeine was assessed. We found that the ORAC_{hydro} was 145 µmol TE/g and the ORACₗᵢₚₒ was 66 µmol TE/g, whereas pure caffeine was associated with an extremely low ORAC_{hydro} (6 µmol TE/g) and ORACₗᵢₚₒ (0 µmol TE/g) (Table 2).

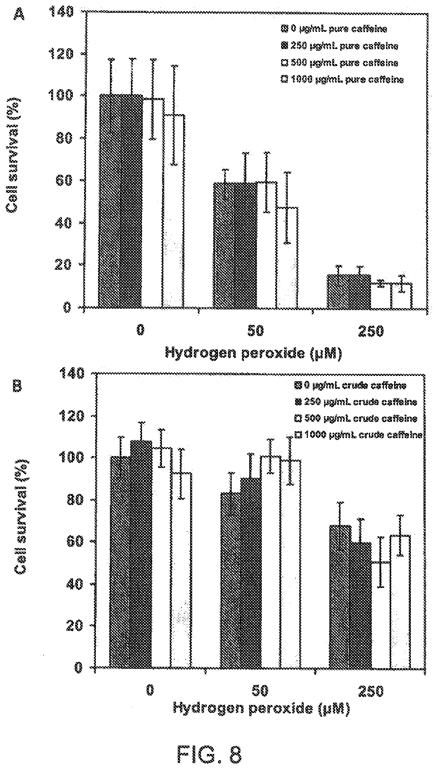

A biological correlate of the crude caffeine's *in vitro* antioxidant activity was evaluated. Primary cortical neuron cells were pretreated with crude caffeine before H₂O₂-induced oxidative stress. FIG. 8A shows that H₂O₂ diminished cell survival in a dose-dependent manner. Pure caffeine was not toxic to primary neuron cells, but did not protect against H₂O₂-induced cell death. Similarly, FIG. 8B shows that crude caffeine was not toxic to primary neuron cells, but did not protect against H₂O₂-induced cell death, even though ORAC values of crude caffeine were much higher than those of pure caffeine (Table 2).

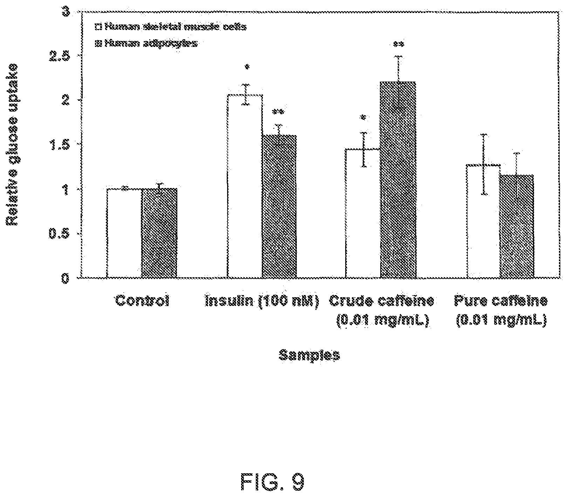

Results for glucose uptake. The anti-hyperglycemic effects of crude caffeine on glucose uptake in human skeletal muscle cells and adipocytes were determined, using insulin as a positive control. In human skeletal muscle cells, 100 nM insulin stimulated a 2.06-fold increase in glucose uptake, and although less effective than insulin, 0.01 mg/mL crude caffeine increased glucose uptake significantly by 1.45 fold (FIG. 9). In contrast, 0.01 mg/mL pure caffeine produced no significant effect. Surprisingly, in human adipocytes, crude caffeine stimulated glucose uptake more effectively than insulin: 0.01 mg/mL crude caffeine induced a 2.20-fold increase in glucose uptake, whereas 100 nM insulin induced a 1.6-fold increase. Pure caffeine (0.01 mg/mL) did not exert a significant effect on glucose uptake. Data were analyzed by one-way ANOVA followed by Tukey's post hoc test for multiple comparisons. Results are expressed as mean ± SD. *P < 0.05 compared with untreated human skeletal muscle cells; **P < 0.05 compared with untreated human adipocytes. These data indicate that crude caffeine stimulates glucose uptake in human fat and muscle cells.

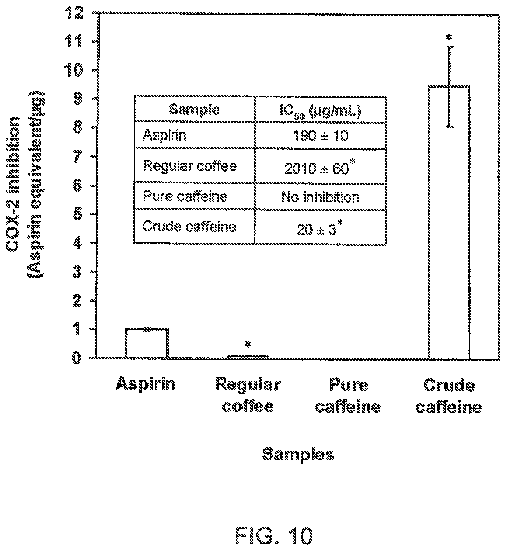

Results for COX-2 inhibition. The anti-inflammatory activity of crude caffeine was evaluated by determining its effect on the inflammatory enzyme COX-2, using aspirin as a positive control. Crude caffeine inhibited COX-2 activity; the IC₅₀ value was 20 µg/mL, which was 9.5 times lower than that of aspirin (IC₅₀, 190 µg/mL) (FIG. 10), indicating that crude caffeine is a more potent COX-2 inhibitor than aspirin. In contrast, the activity of regular coffee (IC₅₀, 2010 µg/mL) was about 10 times lower than that of aspirin. Pure caffeine did not inhibit COX-2 activity. Data were analyzed by one-way ANOVA followed by Tukey's post hoc test for multiple comparisons. Results are expressed as mean ± SD. *P < 0.05 compared with aspirin. These data indicate that crude caffeine inhibits the inflammatory enzyme COX-2.

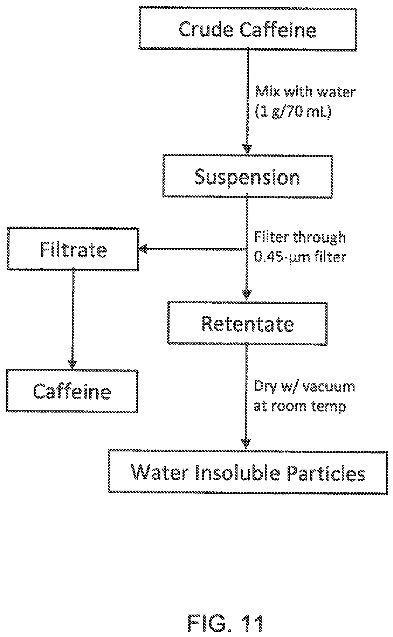

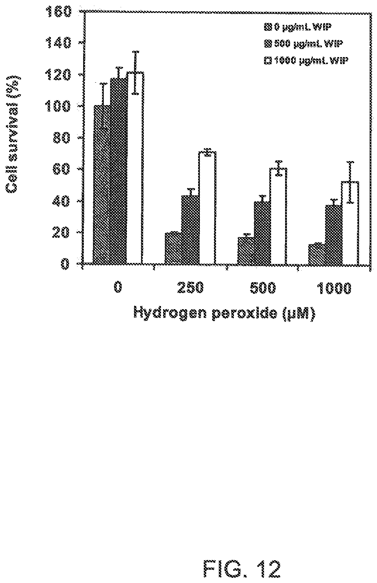

Results for non-caffeine bioactives in crude caffeine. We observed that mixing crude caffeine (1 g) and water (70 mL) produced an aqueous suspension of fine, red-brown water-insoluble particles (WIP), which could be isolated by filtration (FIG. 11). The WIP fraction contained only 1.6% caffeine, about 60 times less than crude caffeine (Table 2). We determined the presence of phenolics in the WIP fraction using the Folin-Ciocalteu assay, and found that the level of total phenolics was 313 mg CE/g (Table 2), or approximately 30% of the WIP fraction by mass. We found that the ORAC_{hydro} was 1321 µmol TE/g and the ORACₗᵢₚₒ was 332 µmol TE/g, about 9 and 5 times higher, respectively, than the values of crude caffeine (Table 2). Further, WIP contained approximately 21% fat, which was the primary reason that it was suspended rather than dissolved in water. Importantly, we evaluated the antioxidant activity of WIP in mouse primary neuron cells. We found that WIP showed no toxicity and significantly ameliorated cell death in H₂O₂-initiated oxidative stress in a dose-dependent manner (FIG. 12). WIP (1000 µg/mL) increased survival of mouse primary neuron cells treated with H₂O₂ (250-1000 µM) by about 3.5-fold. These data indicate that isolation of WIP reduces caffeine and enriches polyphenols, besides concentrating antioxidants and potentiating neuroprotectant activity.

Results for activity-guided fractionation and identification of bioactive compounds. We performed antioxidant activity-guided fractionation and LC-MS analysis of WIP. WIP was separated into 17 fractions by flash chromatography on a C-18 column, and the ORAC_{hydro} of each fraction was determined (Table 3). Fractions 4 and 5 demonstrated the highest antioxidant capacities: 26.4% and 41.0%, respectively, of the total ORAC_{hydro} (Table 3); and were therefore submitted to LC-MS analysis.

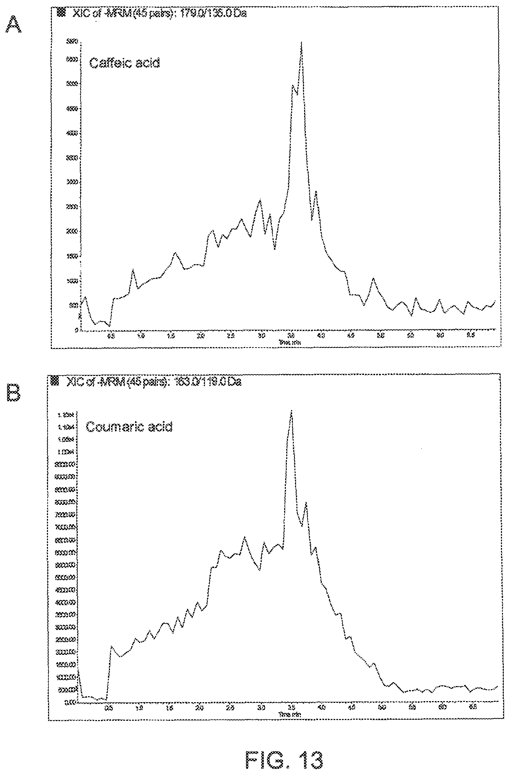

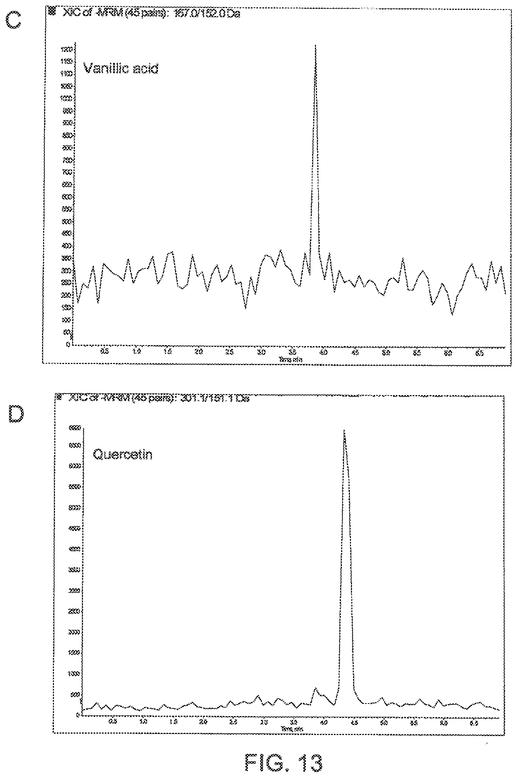

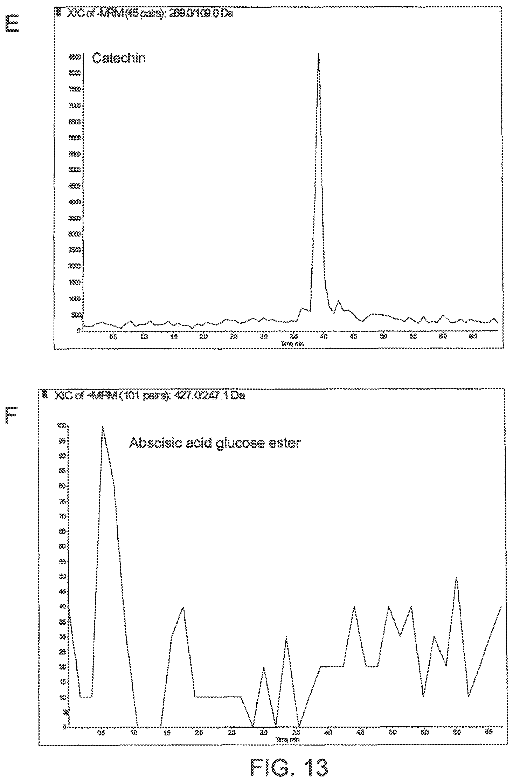

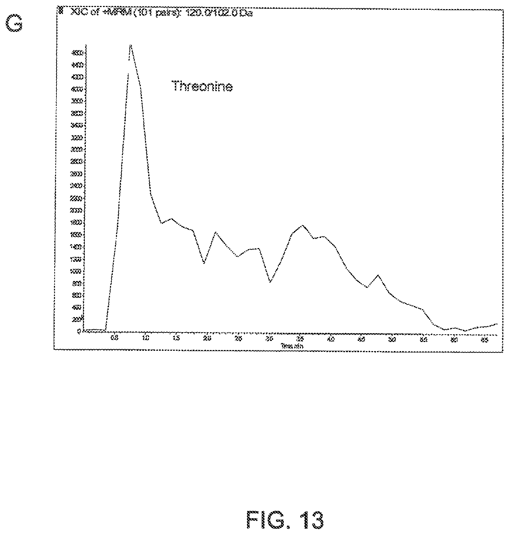

Seven chemicals were identified in both fractions (Table 4): caffeic acid, coumaric acid, vanillic acid, quercetin, catechin, abscisic acid glucose ester, and threonine (FIG. 13).

Results for synergistic effects. We determined the synergistic effects of crude caffeine and WIP when combined with other biological antioxidants. Using an ORAC assay adapted for the linoleate microemulsion system (Sim, W. L. S.; et al., Journal of Agricultural and Food Chemistry 2009, 57, 3409-3414), we determined the antioxidant activity of a-tocopherol (a form of vitamin E) combined with crude caffeine, WIP, ascorbic acid, catechin, chlorogenic acid, WTP + catechin, or WIP + chlorogenic acid (Table 5). Data were expressed as relative ORAC: a relative ORAC of 100% means that the antioxidant activity of the mixture equaled the sum of each component's antioxidant activity (additive effect), whereas a relative ORAC greater than 100% means that the antioxidant activity of the mixture was greater than the sum of each component's antioxidant activity (synergistic effect). We found that crude caffeine and ascorbic acid produced an additive antioxidant effect in combination with α-tocopherol (Table 5). In contrast, WIP, catechin, and chlorogenic acid produced a synergistic effect in combination with α-tocopherol. These data indicate that WIP manifest synergistic effects when used in combination with α-tocopherol.

These examples and embodiments are illustrative and are not to be read as limiting the scope of the invention as it is defined by this specification and the appended claims.

**Table 1. Proximate composition of crude caffeine.**

| Components | Mass (%) |
|---|---|
| Caffeine | 95.95 |
| Moisture | 1.10 |
| Fat | 1.04 |
| Ash | 0.1 |
| Total dietary fiber | < 0.47 |
| Soluble dietary fiber | < 0.47 |
| Insoluble dietary fiber | < 0.47 |
| Protein | < 0.01 |
| Fructose | < 0.10 |
| Glucose | < 0.10 |
| Sucrose | < 0.10 |
| Lactose | < 0.10 |
| Maltose | < 0.10 |

**Table 2. Comparison between caffeine, crude caffeine and crude caffeine water insoluble particles (WIP).**

| | Caffeine | Crude Caffeine | WIP |
|---|---|---|---|
| Caffeine (%) | 100.0 | 95.6 | 1.6 |
| Fat (%) | 0.00 | 1.04 | 1.60 |
| ORAC_{hydro} (µmol TE/g) | 6 | 145 | 1321 |
| ORACₗᵢₚₒ (µmol TE/g) | 0 | 66 | 332 |
| Total phenolics (mg CE/g) | 0 | 10 | 313 |
| Total phenolics (%) | 0 | 1 | 31.3 |
| Ratio of Total phenolics (%) to Caffeine (%) | 0 | <1 | 20 |

| | | | |
|---|---|---|---|
| TE, trolox equivalent; CE, chlorogenic acid equivalent. | | | |

**Table 3. Antioxidant activity of crude caffeine fractions separated by flash chromatography.**

| Fraction # | ORAC_{hydro} (µmol TE/g) | Weight (mg) | TE (µmol) | TE (%) |
|---|---|---|---|---|
| 1 | ND | ND | ND | ND |
| 2 | ND | ND | ND | ND |
| 3 | 1975 | 0.3 | 0.59 | 2.1 |
| 4 | 847 | 8.6 | 7.29 | 26.4 |
| 5 | 2097 | 5.4 | 11.32 | 41.0 |
| 6 | ND | ND | ND | ND |
| 7 | ND | ND | ND | ND |
| 8 | ND | ND | ND | ND |
| 9 | 782 | 0.4 | 0.31 | 1.1 |
| 10 | 1350 | 2.1 | 2.84 | 10.3 |
| 11 | 827 | 2.6 | 2.15 | 7.8 |
| 12 | 1237 | 0.8 | 0.99 | 3.6 |
| 13 | 1290 | 0.5 | 0.65 | 2.4 |
| 14 | 554 | 1.0 | 0.55 | 2.0 |
| 15 | 651 | 0.6 | 0.39 | 1.4 |
| 16 | 1818 | 0.2 | 0.36 | 1.3 |
| 17 | 989 | 0.2 | 0.20 | 0.7 |

| | | | | |
|---|---|---|---|---|
| TE, trolox equivalent; ORAC, oxygen radical absorbance capacity; ND, not detectable. | | | | |

**Table 4. Mass spectroscopy identification of antioxidants in fractions 4 and 5.**

| Name | Structure | Molecular Weight | Molecular Ion ([M-H]⁻) | Fragmentation |
|---|---|---|---|---|
| Caffeic acid | 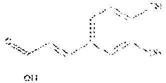 | 180.16 | 179 | [M-H-CO₂]⁻ = 135 |
| Coumaric acid | 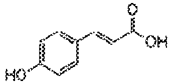 | 164.16 | 163 | [M-H-CO₂]⁻ = 119 |
| Vanillic acid | 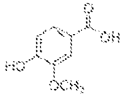 | 168.15 | 167 | [M-H-CH₃]⁻ = 152 |
| Quercetin | 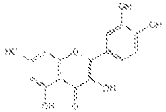 | 302 | 301 | [Retrocyclization product (179)-CO]⁻ = 151 |
| Catechin | 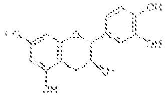 | 290 | 289 | [M-H-C₉H₈O₄]⁻ = 109 |
| Abscisic acid glucose ester | 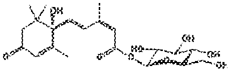 | 428 | 427 | [M-H-C₆H₁₂O₆]⁻ = 247 |
| Threonine | 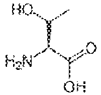 | 121 | 121 | [M-H-H₂O]⁻ = 102 |

**Table 5. Antioxidant activity of combined antioxidants quantitated by relative oxygen radical absorbance capacities.**

| Description | Relative ORAC (%) | Effect |
|---|---|---|
| Crude caffeine + α-tocopherol | 100 | Additive |
| WIP + α-tocopherol | 126 | Synergistic |
| Ascorbic acid + α-tocopherol | 100 | Additive |
| Catechin + α-tocopherol | 166 | Synergistic |
| Chlorogenic acid + α-tocopherol | 168 | Synergistic |
| WIP + catechin + α-tocopherol | 100 | Additive |
| WIP + chlorogenic acid + α-tocopherol | 100 | Additive |
| WIP + catechin | 100 | Additive |
| WIP + chlorogenic acid | 100 | Additive |

| | | |
|---|---|---|
| ORAC, oxygen radical absorbance capacities; WIP, water insoluble particles. | | |

## Claims

1. A process for producing a food product comprising either supplementing a food with, or supplementing a component of a food with, a phytochemical fraction recovered from a crude caffeine, whereby said food product is produced;
wherein said phytochemical fraction has a ratio of polyphenols to caffeine of about 10 - 40 to 1,
wherein said phytochemical fraction is a retentate of a filtration process of a water suspension of crude caffeine, and
wherein said crude caffeine is a product of a green coffee bean supercritical carbon dioxide (scCO₂) decaffeination process.

2. A composition of matter comprising a food product, said food product comprised of a phytochemical fraction recovered from a crude caffeine, said phytochemical fraction having a ratio of polyphenols to caffeine of about 10 - 40 to 1,
wherein said phytochemical fraction is obtainable as a retentate of a filtration process of a water suspension of crude caffeine, and
wherein said crude caffeine is a product of a green coffee bean supercritical carbon dioxide (scCO₂) decaffeination process.

3. The process of claim 1 or the composition of claim 2, wherein the ratio of polyphenols to caffeine is about 10 - 30 to 1.

4. The process of claim 1 or the composition of claim 2, wherein the ratio of polyphenols to caffeine is about 20 to 1.

5. The process of claim 1 or the composition of claim 2" wherein the crude caffeine has an oxygen radical absorbance capacity (ORAC_{hydro} and ORACₗᵢₚₒ) greater than pure caffeine.

6. The process or composition of claim 5" wherein the crude caffeine has an ORAC_{hydro} of 145 mmol TE/g and an ORACₗᵢₚₒ of 66 mmol TE/g.

7. The process or composition of claim 6, wherein the crude caffeine comprises about 1 wt. % phenolics.

8. The process of claim 1, comprising forming a suspension of the crude caffeine and collecting water-insoluble particles (WIP) of the crude caffeine by filtration.

9. The process of claim 8, wherein the WIP comprises caffeic acid, coumaric acid, vanillic acid, quercetin, catechin, abscisic acid glucose ester, and threonine.

10. The composition of claim 2, wherein the phytochemical fraction comprises a water-insoluble particles (WIP) fraction of crude caffeine comprising caffeic acid, coumaric acid, vanillic acid, quercetin, catechin, abscisic acid glucose ester, and threonine.

11. A composition of matter comprising a food product as defined in any of claims 2-7, or 10, for use in the treatment of diabetes mellitus, inhibition of inflammation, dementia, osteoarthritis, rheumatoid arthritis, or age related cognitive decline.

## Patentansprüche

1. Verfahren zur Herstellung eines Nahrungsprodukts umfassend entweder das Supplementieren eines Nahrungsmittels oder das Supplementieren einer Nahrungsmittelkomponente mit einer phytochemischen Fraktion, die aus Rohkoffein erhalten wurde, unter Herstellung des Nahrungsprodukts;
worin die phytochemische Fraktion ein Verhältnis von Polyphenolen zu Koffein von ungefähr 10-40 zu 1 aufweist,
worin die phytochemische Fraktion ein Retentat eines Filtrierprozesses einer Wassersuspension von Rohkoffein ist, und
worin das Rohkoffein ein Produkt eines Entkoffeinierungsverfahrens von grünen Kaffeebohnen unter Verwendung von superkritischem Kohlendioxid (scCO₂) ist.

2. Zusammensetzung umfassend ein Nahrungsprodukt, wobei das Nahrungsprodukt eine phytochemische Fraktion umfasst, welche aus Rohkoffein erhalten wurde, wobei die phytochemische Fraktion ein Verhältnis von Polyphenolen zu Koffein von 10-40 zu 1 aufweist,
worin die phytochemische Fraktion erhältlich ist als Retentat eines Filtrationsverfahrens einer Wassersuspension von Rohkoffein, und
worin das Rohkoffein ein Produkt eines Entkoffeinierungsverfahrens von grünen Kaffeebohnen unter Verwendung von superkritischem Kohlendioxid (scCO₂) ist.

3. Verfahren nach Anspruch 1 oder Zusammensetzung nach Anspruch 2, worin das Verhältnis von Polyphenolen zu Koffein ungefähr 10-30 zu 1 beträgt.

4. Verfahren nach Anspruch 1 oder Zusammensetzung nach Anspruch 2, worin das Verhältnis von Polyphenolen zu Koffein ungefähr 20 zu 1 beträgt.

5. Verfahren nach Anspruch 1 oder Zusammensetzung nach Anspruch 2, worin das Rohkoffein eine größere Sauerstoffradikalabsorptionskapazität (ORAC_{hydro} und ORACₗᵢₚₒ) als reines Koffein aufweist.

6. Verfahren oder Zusammensetzung nach Anspruch 5, worin das Rohkoffein ein ORAC_{hydro} von 145 mmol TE/g und ein ORACₗᵢₚₒ von 66 mmol TE/g aufweist.

7. Verfahren oder Zusammensetzung nach Anspruch 6, worin das Rohkoffein ungefähr 1 Gew.% Phenole aufweist.

8. Verfahren nach Anspruch 1 umfassend das Bilden einer Suspension des Rohkoffeins und das Sammeln wasserunlöslicher Partikel (WIP) aus dem Rohkoffein durch Filtration.

9. Verfahren nach Anspruch 8, worin die WIP Koffeinsäure, Cumarsäure, Vanillinsäure, Quercetin, Katechin, Abscisinsäureglukoseester und Threonin umfassen.

10. Zusammensetzung von Anspruch 2, worin die phytochemische Fraktion eine Fraktion von wasserunlöslichen Partikeln (WIP) von Rohkoffein, umfassend Koffeinsäure, Cumarsäure, Vanillinsäure, Quercetin, Katechin, Abscisinsäureglukoseester und Threonin, umfasst.

11. Zusammensetzung umfassend ein Nahrungsprodukt gemäß einem der Ansprüche 2 bis 7 oder 10 zur Verwendung bei der Behandlung von Diabetis mellitus, Hemmung von Entzündung, Demenz, Osteoarthritis, rheumatoider Arthritis oder altersbedingtem Verfall kognitiver Fähigkeiten.

## Revendications

1. Procédé de production d'un produit alimentaire comprenant le fait d'enrichir un aliment, ou d'enrichir un composant d'un aliment en une fraction phytochimique récupérée de caféine brute, moyennant quoi ledit produit alimentaire est produit ;
dans lequel ladite fraction phytochimique a un rapport de polyphénols sur caféine d'environ 10 à 40 sur 1,
dans lequel ladite fraction phytochimique est un rétentat d'un procédé de filtration d'une suspension aqueuse de caféine brute, et
dans lequel ladite caféine brute est un produit d'un procédé de décaféination par dioxyde de carbone supercritique (scCO₂) de grains de café vert.

2. Composition de matière comprenant un produit alimentaire, ledit produit alimentaire comprenant une fraction phytochimique récupéré de caféine brute, ladite fraction phytochimique ayant un rapport de polyphénols sur caféine d'environ 10 à 40 sur 1,
dans laquelle ladite fraction phytochimique peut être obtenue en tant que rétentat d'un procédé de filtration d'une suspension aqueuse de caféine brute, et
dans laquelle ladite caféine brute est un produit d'un procédé de décaféination par dioxyde de carbone supercritique (scCO₂) de grains de café vert.

3. Procédé de la revendication 1 ou composition de la revendication 2, où le rapport de polyphénols sur caféine est d'environ 10 à 30 sur 1.

4. Procédé de la revendication 1 ou composition de la revendication 2, où le rapport de polyphénols sur caféine est d'environ 20 sur 1.

5. Procédé de la revendication 1 ou composition de la revendication 2, où la caféine brute a une capacité d'absorption des radicaux d'oxygène (ORAC_{hydro} et ORACₗᵢₚₒ) supérieure à celle de la caféine pure.

6. Procédé ou composition de la revendication 5, où la caféine brute a une ORAC_{hydro} de 145 mmol TE/g et une ORACₗᵢₚₒ de 66 mmol TE/g.

7. Procédé ou composition de la revendication 6, où la caféine brute comprend environ 1 % en poids de dérivés phénoliques.

8. Procédé de la revendication 1, comprenant le fait de former une suspension de la caféine brute et de recueillir des particules insolubles dans l'eau (WIP) de la caféine brute par filtration.

9. Procédé de la revendication 8, dans lequel les particules WIP comprennent de l'acide caféique, de l'acide coumarique, de l'acide vanillique, de la quercétine, de la catéchine, de l'ester de glucose d'acide abscissique et de la thréonine.

10. Composition de la revendication 2, dans laquelle la fraction phytochimique comprend une fraction de particules insolubles dans l'eau (WIP) de caféine brute comprenant de l'acide caféique, de l'acide coumarique, de l'acide vanillique, de la quercétine, de la catéchine, de l'ester de glucose d'acide abscissique et de la thréonine.

11. Composition de matière comprenant un produit alimentaire telle que définie dans l'une des revendications 2 à 7 ou 10, pour une utilisation dans le traitement de diabète sucré, l'inhibition d'inflammation, le traitement de la démence, de l'ostéo-arthrite, de la polyarthrite rhumatoïde ou du déclin cognitif lié à l'âge.
